(19) Europäisches Patentamt
European Patent Office
Office européen des brevets

(11) EP 2 288 671 B1

(12) EUROPÄISCHE PATENTSCHRIFT

(45) Veröffentlichungstag und Bekanntmachung des Hinweises auf die Patenterteilung:
**15.08.2012 Patentblatt 2012/33**

(21) Anmeldenummer: **09761713.8**

(22) Anmeldetag: **09.06.2009**

(51) Int Cl.:
***C09K 11/06*** *(2006.01)*

(86) Internationale Anmeldenummer:
**PCT/EP2009/057088**

(87) Internationale Veröffentlichungsnummer:
**WO 2009/150150 (17.12.2009 Gazette 2009/51)**

# (54) NEUE ÜBERGANGSMETALL-KOMPLEXE UND DEREN VERWENDUNG IN ORGANISCHEN LEUCHTDIODEN - III

NOVEL TRANSITION METAL COMPLEX AND USE THEREOF IN ORGANIC LIGHT-EMITTING DIODES - III

NOUVEAUX COMPLEXES DE MÉTAUX DE TRANSITION ET LEUR UTILISATION DANS DES DIODES ÉLECTROLUMINESCENTES ORGANIQUES III

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **10.06.2008 EP 08157949**

(43) Veröffentlichungstag der Anmeldung:
**02.03.2011 Patentblatt 2011/09**

(73) Patentinhaber: **BASF SE**
**67056 Ludwigshafen (DE)**

(72) Erfinder:
- **MOLT, Oliver**
  **69493 Hirschberg (DE)**
- **LENNARTZ, Christina**
  **67105 Schifferstadt (DE)**
- **FUCHS, Evelyn**
  **68199 Mannheim (DE)**
- **DORMANN, Korinna**
  **67098 Bad Dürkheim (DE)**
- **LANGER, Nicolle**
  **64646 Heppenheim (DE)**
- **SCHILDKNECHT, Christian**
  **68305 Mannheim (DE)**
- **RUDOLPH, Jens**
  **67547 Worms (DE)**
- **WAGENBLAST, Gerhard**
  **67157 Wachenheim (DE)**
- **WATANABE, Soichi**
  **68161 Mannheim (DE)**

(56) Entgegenhaltungen:
**EP-A- 1 577 300**
**WO-A-2008/054024**
**WO-A-2008/117889**
**WO-A1-2008/156879**
**WO-A2-2007/095118**
**WO-A2-2009/085344**
**US-A1- 2005 171 076**

- **PENG XIE: "Structure-Based Design of an Organoruthenium Phosphatidyl-inositol-3-kinase Inhibitor Reveals a Swith Governing Lipid Kinase Potency and Selectivity" ACS CHEMICAL BIOLOGY, Bd. 3, Nr. 5, 16. Mai 2008 (2008-05-16), Seiten 305-315, XP002544904 USA**
- **HOWARD BREGMAN: "Rapid Access to Unexplored Chemical Space by Ligand Scanning around a Ruthenium Center" JOURNAL OF THE AMERICAN CHEMICAL SOCIETY, Bd. 128, 22. Dezember 2005 (2005-12-22), Seiten 877-884, XP002544905 USA**
- **DOUGLAS S. WILLIAMS: "Platinum Complex as a Nanomolar Protein Kinase Inhibitor" INORGANIC CHEMISTRY, Bd. 46, 22. März 2007 (2007-03-22), Seiten 2944-2946, XP002544906 USA**
- **HOWARD BREGMAN: "Ruthenium Half-Sandwich Complexes as Protein Kinase Inhibitors" ORGANIC LETTERS, Bd. 8, Nr. 24, 4. November 2006 (2006-11-04), Seiten 5465-5468, XP002544907 USA**
- **JUDIT É. DEBRECZENI: "Ruthenium Half-Sandwich Complexes Bound to Protein Kinase Pim-1" ANGEWANDTE CHEMIE INTERNATIONAL EDITION, Bd. 45, 2006, Seiten 1580-1585, XP002544908 Weinheim**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 288 671 B1

- HOWARD BREGMAN: "Pyrido[2,3-a]pyrrolo [3,4,c]carbazole-5,7( 6H)diones" SYNTHESIS, Nr. 9, 21. April 2005 (2005-04-21), Seiten 1521-1527, XP002544909 New York
- JASNA MAKSIMOSKA: "Similar Biological Activities of Two Isostructural Ruthenium and Osmium Complexes" CHEMISTRY, Bd. 14, 29. Mai 2008 (2008-05-29), Seiten 4816-4822, XP002544910 Weinheim
- PENG XIE ET AL: "Structure-Based Design of an Organoruthenium Phosphatidyl-inositol-3-kinase Inhibitor Reveals a Swith Governing Lipid Kinase Potency and Selectivity", ACS CHEMICAL BIOLOGY, AMERICAN CHEMICAL SOCIETY, WASHINGTON, DC, US, vol. 3, no. 5, 16 May 2008 (2008-05-16), pages 305-315, XP002544904, ISSN: 1554-8929, DOI: DOI:10.1021/CB800039Y [retrieved on 2008-05-16]

## Beschreibung

Die vorliegende Erfindung betrifft Metallkomplexe enthaltend mindestens einen polycyclischen aromatischen Liganden, der über zwei Stickstoffatome mit dem Zentralmetall verknüpft ist, eine organische Leuchtdiode enthaltend mindestens einen erfindungsgemäßen Metallkomplex, eine Licht-emittierende Schicht enthaltend mindestens einen erfindungsgemäßen Metallkomplex, eine organische Leuchtdiode enthaltend mindestens eine erfindungsgemäße Licht-emittierende Schicht, die Verwendung des mindestens einen erfindungsgemäßen Metallkomplexes in organischen Leuchtdioden sowie eine Vorrichtung ausgewählt aus der Gruppe bestehend aus stationären Bildschirmen, wie Bildschirmen von Computern, Fernsehern, Bildschirmen in Druckern, Küchengeräten sowie Reklametafeln, Beleuchtungen, Hinweistafeln und mobilen Bildschirmen, wie Bildschirmen in Handys, Laptops, Digitalkameras, Fahrzeugen sowie Zielanzeigen an Bussen und Bahnen, enthaltend mindestens eine erfindungsgemäße organische Leuchtdiode.

In organischen Leuchtdioden (OLED) wird die Eigenschaft von Materialien ausgenutzt, Licht zu emittieren, wenn sie durch elektrischen Strom angeregt werden. OLEDs sind insbesondere interessant als Alternative zu Kathodenstrahlröhren und Flüssigkristalldisplays zur Herstellung von Flachbildschirmen. Aufgrund der sehr kompakten Bauweise und des intrinsisch niedrigen Stromverbrauchs eignen sich die Vorrichtungen enthaltend OLEDs insbesondere für mobile Anwendungen, z. B. für Anwendungen in Handys, Laptops, usw.

Die Grundprinzipien der Funktionsweise von OLEDs sowie geeignete Aufbauten (Schichten) von OLEDs sind z. B. in WO 2005/113704 und deren zitierten Literatur genannt.

Als Licht-emittierende Materialien (Emitter) können neben fluoreszierenden Materialien (Fluoreszenz-Emitter) phosphoreszierende Materialien (Phosphoreszenz-Emitter) eingesetzt werden. Bei den Phosphoreszenz-Emittern handelt es sich üblicherweise um metallorganische Komplexe, die im Gegensatz zu den Fluoreszenz-Emittern, die eine Singulett-Emission zeigen, eine Triplett-Emission zeigen (M. A. Baldo et al., Appl. Phys. Lett. 1999, 75, 4 - 6). Aus quantenmechanischen Gründen ist bei Verwendung der Phosphoreszenz-Emitter eine bis zu vierfache Quanten-, Energie- und Leistungseffizienz möglich. Um die Vorteile des Einbaus der metallorganischen Phosphoreszenz-Emitter in die Praxis umzusetzen, ist es erforderlich, Phosphoreszenz-Emitter bereitzustellen, die eine hohe operative Lebensdauer, eine hohe Effizienz, eine hohe Stabilität gegenüber Temperaturbelastung und eine niedrige Einsatz- und Betriebsspannung aufweisen.

Um den vorstehend genannten Anforderungen zu genügen, sind zahlreiche Phosphoreszenz-Emitter im Stand der Technik vorgeschlagen worden.

So betrifft WO 2007/095118 Metallkomplexe cyclometallierter Imidazo[1,2-f]phenanthridin- und Diimidazo[1,2-A:1',2'-C]chinazolinliganden sowie isoelektronischer und benzanellierter Derivate davon. Die Metallkomplexe gemäß WO 2007/095118 zeichnen sich dadurch aus, dass die vorstehend genannten Liganden gemäß der Offenbarung in WO 2007/095118 als Heteroatome im Wesentlichen ausschließlich Stickstoffatome enthalten. Die Metallkomplexe sind phosphoreszierend und werden in OLEDs eingesetzt. Die OLEDs zeigen gemäß WO 2007/095118 eine langlebige und effiziente blaue, grüne und rote Emission.

Aufgabe der vorliegenden Erfindung gegenüber dem vorstehend genannten Stand der Technik ist es, weitere zur Phosphoreszenz geeignete Metallkomplexe für die Verwendung in OLEDs bereitzustellen, die ein ausgewogenes Eigenschaftsspektrum zeigen, z. B. gute Effizienzen, eine verbesserte Lebensdauer und höhere Stabilitäten im Device sowie gute Ladungstransporteigenschaften und thermische Stabilität, und die Emission im sichtbaren Bereich des elektromagnetischen Spektrums, bevorzugt im blauen bis hellblauen Bereich des elektromagnetischen Spektrums, bei Einsatz in einer OLED als Emittermaterial Elektrolumineszenz zeigen.

Diese Aufgabe wird gelöst durch einen Metallkomplex, enthaltend mindestens einen Liganden der allgemeinen Formel (I)

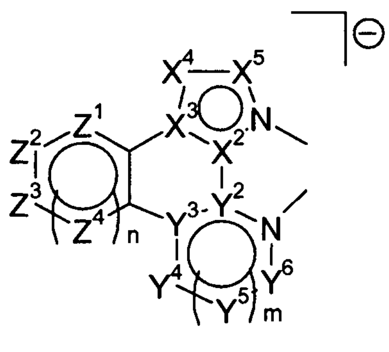

(I)

worin die Symbole die folgenden Bedeutungen aufweisen:

| | |
|---|---|
| $Y^5$, $Z^4$ | unabhängig voneinander $CR^1$, CH oder N; |
| $X^2$, $X^3$, $Y^2$, $Y^3$ | unabhängig voneinander N oder C; |
| $X^4$, $X^5$ | unabhängig voneinander $CR^1$, CH oder N; |
| $Y^4$, $Y^6$ | unabhängig voneinander $CR^1$, CH oder N wobei $Y^4$ oder $Y^6$ im Falle von m = 0 des Weiteren $NR^2$, S oder O bedeuten können; |
| $Z^1$ $Z^2$, $Z^3$ | unabhängig voneinander $CR^1$, CH oder N; wobei $Z^1$, $Z^2$ oder $Z^3$ im Falle von n = 0 des Weiteren $NR^2$, S oder O bedeuten können; |
| $R^1$ | unabhängig voneinander unsubstituiertes oder substituiertes Alkyl, unsubstituiertes oder substituiertes Cycloalkyl, unsubstituiertes oder substituiertesHeterocycloalkyl, unsubstituiertes oder substituiertes Aryl, unsubstituiertes oder substituiertes Heteroaryl, unsubstituiertes oder substituiertes Alkenyl, unsubstituiertes oder substituiertes Cycloalkenyl, unsubstituiertes oder substituiertes Alkinyl, $SiR^3_3$, Halogen, ein Substituent mit Donor- oder Akzeptorwirkung; des Weiteren können zwei Reste $R^1$ gemeinsam eine Alkylen- oder Arylenbrücke bilden; |
| $R^2$ | unabhängig voneinander unsubstituiertes oder substituiertes Alkyl, unsubstituiertes oder substituiertes Aryl oder unsubstituiertes oder substituiertes Heteroaryl; des Weiteren können zwei Reste $R^2$ oder ein Rest $R^2$ und ein Rest $R^1$ gemeinsam eine Alkylen- oder Arylenbrücke bilden; |
| $R^3$ | unabhängig voneinander unsubstituiertes oder substituiertes Alkyl, unsubstituiertes oder substituiertes Aryl oder unsubstituiertes oder substituiertes Heteroaryl; |
| m | 0 oder 1, wobei die Gruppen $Y^5$ bei m = 0 nicht vorhanden ist; |
| n | 0 oder 1, wobei die Gruppen $Z^4$ bei n = 0 nicht vorhanden ist. |

Es wurde gefunden, dass für die Verwendung in OLEDs geeignete Metallkomplexe bereitgestellt werden können, wobei sich die OLEDs durch ein ausgewogenes Eigenschaftsspektrum, z.B. durch gute Effizienzen, eine hervorragende Lebensdauer und sehr gute Stabilitäten im Device sowie gute Ladungstransporteigenschaften und thermische Stabilität gegenüber im Stand der Technik bekannten OLEDs auszeichnen. Es können bei Verwendung der erfindungsgemäßen Metallkomplexe OLEDs bereitgestellt werden, die im sichtbaren Bereich des elektromagnetischen Spektrums, insbesondere blauen bis hellblauen Bereich des elektromagnetischen Spektrums, Licht emittieren.

Die erfindungsgemäßen Metallkomplexe können in jeder Schicht einer OLED eingesetzt werden, wobei das Ligandgerüst oder Zentralmetall zur Anpassung an gewünschte Eigenschaften der Metallkomplexe variiert werden kann. Beispielsweise ist der Einsatz der erfindungsgemäßen Metallkomplexe in der Licht-emittierenden Schicht, einer Blockschicht für Elektronen, einer Blockschicht für Excitonen, einer Blockschicht für Löcher, einer Lochtransportschicht und/oder einer Elektronentransportschicht der OLED in Abhängigkeit von dem Substitutionsmuster der erfindungsgemäßen Metallkomplexe und den elektronischen Gegebenheiten in weiteren in der OLED vorliegenden Schichten möglich. Bevorzugt werden die erfindungsgemäßen Metallkomplexe in der Licht-emittierenden Schicht eingesetzt. Darin können die erfindungsgemäßen Metallkomplexe als Emittermaterialien und/oder Matrixmaterialien eingesetzt werden. Bevorzugt werden die erfindungsgemäßen Metallkomplexe als Emittermaterialien in OLEDs eingesetzt.

Im Sinne der vorliegenden Anmeldung haben die Begriffe unsubstituierter oder substituierter Arylrest oder -gruppe, unsubstituierter oder substituierter Heteroarylrest oder - gruppe, unsubstituierter oder substituierter Alkylrest oder -gruppe, unsubstituierter oder substituierter Cycloalkylrest oder -gruppe, unsubstituierter oder substituierter Heterocycloalkylrest oder -gruppe, unsubstituierter oder substituierter Alkenylrest oder - gruppe, unsubstituierter oder substituierter Alkinylrest oder -gruppe, Aralkylrest oder - gruppe, und Gruppen mit Donor- und/oder Akzeptorwirkung die folgenden Bedeutungen:

Unter einem Arylrest (oder -gruppe) ist ein Rest mit einem Grundgerüst von 6 bis 30 Kohlenstoffatomen, bevorzugt 6 bis 18 Kohlenstoffatomen zu verstehen, der aus einem aromatischen Ring oder mehreren kondensierten aromatischen Ringen aufgebaut ist. Geeignete Grundgerüste sind zum Beispiel Phenyl, Naphthyl, Anthracenyl oder Phenanthrenyl. Dieses Grundgerüst kann unsubstituiert sein (d. h., dass alle Kohlenstoffatome, die substituierbar sind, Wasserstoffatome tragen), oder an einer, mehreren oder allen substituierbaren Positionen des Grundgerüsts substituiert sein. Geeignete Substituenten sind zum Beispiel Alkylreste, bevorzugt Alkylreste mit 1 bis 8 Kohlenstoffatomen, besonders bevorzugt Methyl, Ethyl oder i-Propyl, Arylreste, bevorzugt $C_6$-Arylreste, die wiederum substituiert oder unsubstituiert sein können, Heteroarylreste, bevorzugt Heteroarylreste, die mindestens ein Stickstoffatom enthalten, besonders bevorzugt Pyridylreste, Alkenylreste, bevorzugt Alkenylreste, die eine Doppelbindung tragen, besonders bevorzugt Alkenylreste mit einer Doppelbindung und 1 bis 8 Kohlenstoffatomen, oder Gruppen mit Donor- oder Akzeptorwirkung. Geeignete Gruppen, mit Donor- oder Akzeptorwirkung sind nachstehend genannt. Ganz besonders bevorzugt tragen die substituierten Arylreste Substituenten ausgewählt aus der Gruppe bestehend aus Methyl, Isopropyl, F, CN, Aryloxy und Alkoxy, Thioaryl, Thioalkyl, Heteroaryl. Bevorzugt ist der Arylrest oder die Arylgruppe ein $C_6$-$C_{18}$-Arylrest, besonders bevorzugt ein $C_6$-Arylrest, der gegebenenfalls mit mindestens einem oder mehreren der vorstehend genannten Substituenten substituiert ist. Besonders bevorzugt weist der $C_6$-$C_{18}$-Arylrest, bevorzugt $C_6$-Arylrest, keinen, einen, zwei, drei oder vier der vorstehend genannten Substituenten auf.

Unter einem Heteroarylrest oder einer Heteroarylgruppe sind Reste zu verstehen, die sich von den vorstehend genannten Arylresten dadurch unterscheiden, dass in dem Grundgerüst der Arylreste mindestens ein Kohlenstoffatom durch ein Heteroatom ersetzt ist. Bevorzugte Heteroatome sind N, O und S. Ganz besonders bevorzugt sind ein oder zwei Kohlenstoffatome des Grundgerüsts der Arylreste durch Heteroatome ersetzt. Insbesondere bevorzugt ist das Grundgerüst ausgewählt aus Systemen wie Pyridin und fünfgliedrigen Heteroaromaten wie Pyrrol, Furan, Pyrazol, Imidazol, Thiophen, Oxazol, Thiazol, Triazol. Das Grundgerüst kann an einer, mehreren oder allen substituierbaren Positionen des Grundgerüsts substituiert sein. Geeignete Substituenten sind dieselben, die bereits bezüglich der Arylgruppen genannt wurden.

Unter einem Alkylrest oder einer Alkylgruppe ist ein Rest mit 1 bis 20 Kohlenstoffatomen, bevorzugt 1 bis 10 Kohlenstoffatomen, besonders bevorzugt 1 bis 8, ganz besonders bevorzugt 1 bis 4 Kohlenstoffatomen zu verstehen. Dieser Alkylrest kann verzweigt oder unverzweigt sein und gegebenenfalls mit einem oder mehreren Heteroatomen, bevorzugt Si, N, O oder S, besonders bevorzugt N, O oder S, unterbrochen sein. Des Weiteren kann dieser Alkylrest mit einem oder mehreren der bezüglich der Arylgruppen genannten Substituenten substituiert sein. Es ist ebenfalls möglich, dass der Alkylrest eine oder mehrere (Hetero-)-Arylgruppen trägt. Im Sinne der vorliegenden Anmeldung stellen z.B. Benzylreste somit substituierte Alkylreste dar. Dabei sind alle der vorstehend aufgeführten (Hetero-)Arylgruppen geeignet. Besonders bevorzugt sind die Alkylreste ausgewählt aus der Gruppe bestehend aus Methyl, Ethyl, iso-Propyl, n-Propyl, n-Butyl, iso-Butyl und tert-Butyl, ganz besonders bevorzugt sind Methyl, isoPropyl.

Unter einem Cycloalkylrest oder einer Cycloalkylgruppe ist ein Rest mit 3 bis 20 Kohlenstoffatomen, bevorzugt 3 bis 10 Kohlenstoffatomen, besonders bevorzugt 3 bis 8 Kohlenstoffatomen zu verstehen. Dieses Grundgerüst kann unsubstituiert sein (d.h., dass alle Kohlenstoffatome, die substituierbar sind, Wasserstoffatome tragen), oder an einer, mehreren oder allen substituierbaren Positionen des Grundgerüsts substituiert sein. Geeignete Substituenten sind die bereits vorstehend bezüglich der Arylreste genannten Gruppen. Beispiele für geeignete Cycloalkylreste sind Cyclopropyl, Cyclopentyl und Cyclohexyl.

Unter einem Heterocycloalkylrest oder einer Heterocycloalkylgruppe sind Reste zu verstehen, die sich von den vorstehend genannten Cycloalkylresten dadurch unterscheiden, dass in dem Grundgerüst der Cycloalkylreste mindestens ein Kohlenstoffatom durch ein Heteroatom ersetzt ist. Bevorzugte Heteroatome sind N, O und S. Ganz besonders bevorzugt sind ein oder zwei Kohlenstoffatome des Grundgerüsts der Cycloalkylreste durch Heteroatome ersetzt. Beispiele für geeignete Heterocycloalkylreste sind Reste abgeleitet von Pyrrolidin, Piperidin, Piperazin, Tetrahydrofuran, Dioxan.

Unter einem Alkenylrest oder einer Alkenylgruppe ist ein Rest zu verstehen, der den vorstehend genannten Alkylresten mit mindestens zwei Kohlenstoffatomen entspricht, mit dem Unterschied, dass mindestens eine C-C-Einfachbindung des Alkylrests durch eine C-C-Doppelbindung ersetzt ist. Bevorzugt weist der Alkenylrest eine oder zwei Doppelbindungen auf.

Unter einem Alkinylrest oder einer Alkinylgruppe ist ein Rest zu verstehen, der den vorstehend genannten Alkylresten mit mindestens zwei Kohlenstoffatomen entspricht, mit dem Unterschied, dass mindestens eine C-C-Einfachbindung des Alkylrests durch eine C-C-Dreifachbindung ersetzt ist. Bevorzugt weist der Alkinylrest eine oder zwei Dreifachbindungen auf.

Die Begriffe Alkylen und Arylen haben im Sinne der vorliegenden Anmeldung die bezüglich der Alkyl-, und Arylreste genannten Bedeutungen mit dem Unterschied, dass die Alkylen-, und Arylengruppen zwei Bindungsstellen aufweisen.

Bevorzugte Alkylengruppen sind $(CR^4{}_2)_n$, wobei $R^4$ H oder Alkyl, bevorzugt H, Methyl oder Ethyl, besonders bevorzugt H und n 1 bis 3, bevorzugt 1 oder 2, besonders bevorzugt 1 bedeutet. Ganz besonders bevorzugt ist die Alkylengruppe $CH_2$.

Bevorzugte Arylengruppen sind 1,2-, 1,3- oder 1,4-Phenylengruppen, die unsubstituiert sind oder die bezüglich der Arylreste genannten Substituenten tragen können.

Unter einer Gruppe oder einem Substituenten mit Donor- oder Akzeptorwirkung sind im Sinne der vorliegenden Anmeldung die folgenden Gruppen zu verstehen:

Unter Gruppen mit Donorwirkung sind Gruppen zu verstehen, die einen +|- und/oder +M-Effekt aufweisen, und unter Gruppen mit Akzeptorwirkung sind Gruppen zu verstehen, die einen -|- und/oder -M-Effekt aufweisen. Geeignete

Gruppen, mit Donor- oder Akzeptorwirkung sind Halogenreste, bevorzugt F, Cl, Br, besonders bevorzugt F, Alkoxyreste oder Aryloxyreste, $OR^3$, Carbonylreste, Esterreste, sowohl Oxycarbonyl als auch Carbonyloxy, Aminogruppen, $NR^3_2$, Amidreste, $CH_2F$-Gruppen, $CHF_2$-Gruppen, $CF_3$-Gruppen, CN-Gruppen, Thiogruppen, Sulfonsäuregruppen, Sulfonsäureestergruppen, Boronsäuregruppen, Boronsäureestergruppen, Phosphonsäuregruppen, Phosphonsäureestergruppen, Phosphinreste, Sulfoxidreste, Sulfonylreste, Sulfidreste, $SR^3$, Nitrogruppen, OCN, Boranreste, Silylgruppen, $SiR_3^3$, Stannatreste, Iminogruppen, Hydrazinreste, Hydrazonreste, Oximreste, Nitrosogruppen, Diazogruppen, Phosphinoxidgruppen, Hydroxygruppen oder SCN-Gruppen. Ganz besonders bevorzugt sind F, Cl, CN, Aryloxy, Alkoxy, Amino, $CF_3$-Gruppen, Sulfonyl, Silyl, Sulfid und Heteroaryl. Insbesondere ganz besonders bevorzugt sind Heteroaryl, Silyl ($SiR^3_3$), F, Alkoxy oder Aryloxy ($OR^3$), Sulfidreste ($SR^3$), Amino ($NR^3_2$) und CN. Die Reste $R^3$ sind nachstehend definiert.

Die vorstehend genannten Gruppen mit Donor- oder Akzeptorwirkung schließen nicht aus, dass weitere der in der vorliegenden Anmeldung genannte Reste und Substituenten, die nicht in der vorstehenden Liste der Gruppen mit Donor- oder Akzeptorwirkung aufgeführt sind, eine Donor- oder Akzeptorwirkung aufweisen.

Die Arylreste oder -gruppen, Heteroarylreste oder -gruppen, Alkylreste oder -gruppen, Cycloalkylreste oder -gruppen, Heterocycloalkylreste oder -gruppen, Alkenylreste oder -gruppen, Alkinylreste oder -gruppen und Gruppen mit Donor- und/oder Akzeptorwirkung, sowie die Alkylen- und Arylenreste oder -gruppen können - wie vorstehend erwähnt - substituiert oder unsubstituiert sein. Unter einer unsubstituierten Gruppe ist im Sinne der vorliegenden Anmeldung eine Gruppe zu verstehen, worin die substituierbaren Atome der Gruppe Wasserstoffatome tragen. Unter einer substituierten Gruppe ist im Sinne der vorliegenden Anmeldung eine Gruppe zu verstehen, worin ein oder mehrere substituierbare Atom(e) mindestens an einer Position anstelle eines Wasserstoffatoms einen Substituenten tragen. Geeignete Substituenten sind die vorstehend bezüglich der Arylreste oder -gruppen genannten Substituenten.

Kommen Reste mit denselben Nummerierungen mehrfach in den Verbindungen gemäß der vorliegenden Anmeldung vor, so können diese Reste jeweils unabhängig voneinander die genannten Bedeutungen aufweisen.

In einer Ausführungsform der vorliegenden Erfindung können m und n in den Liganden der allgemeinen Formel (I) unabhängig voneinander 0 oder 1 bedeuten, wobei die Gruppen $Z^4$ bzw. $Y^5$ bei n bzw. m = 0 nicht vorhanden sind. Für den Fall von m = 0 ist der aus den Elementen N, $X^2$, $X^3$, $X^4$ und $X^5$ gebildete aromatische 5-Ring somit mit einem aus den Elementen N, $Y^2$, $Y^3$, $Y^4$ und $Y^6$ gebildeten 5-Ring verbunden. Für m = 1 ist der aus N, $X^2$, $X^3$, $X^4$ und $X^5$ gebildete aromatische 5-Ring mit einem aus den Elementen N, $Y^2$, $Y^3$, $Y^4$, $Y^5$ und $Y^6$ gebildeten aromatischen 6-Ring verbunden. Für n = 0 ist der aus N, $X^2$, $X^3$, $X^4$, $X^5$ gebildete aromatische 5-Ring mit einem aus $Z^1$, $Z^2$, $Z^3$ und zwei Kohlenstoffatomen gebildeten aromatischen 5-Ring verbunden und für n = 1 ist der aus N, $X^2$, $X^3$, $X^4$ und $X^5$ gebildete aromatische 5-Ring mit einem aus $Z^1$, $Z^2$, $Z^3$, $Z^4$ und zwei Kohlenstoffatomen gebildeten aromatischen 6-Ring verbunden. Bevorzugt ist m in den Liganden der Formel (I) 1. In einer weiteren bevorzugten Ausführungsform ist n = 1. Besonders bevorzugt sind sowohl n als auch m = 1.

Die Symbole $X^2$, $X^3$, $X^4$, $X^5$, $Y^2$, $Y^3$, $Y^4$, $Y^5$, $Y^6$, $Z^1$, $Z^2$, $Z^3$, $Z^4$ weisen unabhängig voneinander die folgenden Bedeutungen auf:

Für m = 1 und n = 0 oder 1 bedeuten :

| | |
|---|---|
| $X^4$, $X^5$, $Y^4$, $Y^5$, $Y^6$, $Z^1$, $Z^2$, $Z^3$, $Z^4$ | unabhängig voneinander $CR^1$, CH oder N; |
| $X^3$, $Y^3$ | N oder C, bevorzugt C; |
| $X^2$, $Y^2$ | N oder C, bevorzugt C; |

In einer weiteren bevorzugten Ausführungsform weisen die Symbole $Z^1$, $Z^2$, $Z^3$, $Z^4$ in dem Liganden der Formel (I) unabhängig voneinander die folgenden Bedeutungen auf:

$Z^1$, $Z^2$, $Z^3$, $Z^4$ unabhängig voneinander $CR^1$, CH oder N bedeuten, wobei bevorzugt 0, 1 oder 2 Gruppen $Z^1$, $Z^2$, $Z^3$, $Z^4$ N bedeuten.

In einer weiteren bevorzugten Ausführungsform bedeuten $Y^2$, $Y^3$, $Y^4$, $Y^5$ und $Y^6$ in dem Liganden der Formel (I):

$Y^4$, $Y^5$, $Y^6$ unabhängig voneinander CH oder $CR^1$; und

$Y^2$, $Y^3$ C.

In einer weiteren bevorzugten Ausführungsform bedeuten $X^2$, $X^3$, $X^4$ und $X^5$ in dem Liganden der Formel (I):

$X^2$, $X^3$ C; und

$X^4$, $X^5$ unabhängig voneinander N, CH oder $CR^1$.

In einer weiteren Ausführungsform enthält das Grundgerüst des Liganden der Formel (I) neben den beiden N-Atomen 0, 1, 2, 3 oder 4, bevorzugt 0, 1 oder 2 weitere Heteroatome ausgewählt aus N, O oder S, bevorzugt N. Unter dem Grundgerüst des Liganden der Formel (I) ist das Grundgerüst ohne Berücksichtigung der Liganden (Reste $R^1$, $R^2$, $R^3$) an dem Grundgerüst der Formel (I) zu verstehen.

$R^1$ bedeutet in dem Liganden der allgemeinen Formel (I) unabhängig voneinander unsubstituiertes oder substituiertes Alkyl, unsubstituiertes oder substituiertes Cycloalkyl, unsubstituiertes oder substituiertes Heterocycloalkyl, unsubstituiertes oder substituiertes Aryl, unsubstituiertes oder substituiertes Heteroaryl, unsubstituiertes oder substituiertes Alkenyl, unsubstituiertes oder substituiertes Cycloalkenyl, unsubstituiertes oder substituiertes Alkinyl, $SiR^3_3$, Halogen, einen Substituenten mit Donor- oder Akzeptorwirkung, oder zwei Reste $R^1$ können gemeinsam eine gegebenenfalls substituierte Alkylen- oder Arylenbrücke bilden. Dabei können die beiden Reste $R^1$ zu einem einzigen Zyklus der Liganden der allgemeinen Formel (I) oder zu zwei verschiedenen Zyklen des Liganden der allgemeinen Formel (I) gehören. Z.B. können in dem Fall, wenn $X^4$ und $X^5$ $CR^1$ bedeuten, die beiden Reste $R^1$ gemeinsam eine Alkylen- oder Arylenbrücke bilden. Es ist ebenfalls möglich, dass in dem Fall, wenn $Z^1$ und $X^4$ oder $Z^4$ (bei n = 1) bzw. $Z^3$ (bei n = 0) und $Y^4$ $CR^1$ bedeuten, zwei Reste $R^1$ gemeinsam eine Alkylenoder Arylenbrücke bilden. Geeignete und bevorzugte Alkyl-, Cycloalkyl-, Heterocycloalkyl-, Aryl-, Heteroaryl-, Alkenyl-, Cycloalkenyl-, Alkinylgruppen sowie Substituenten mit Donor- oder Akzeptorwirkung und Alkylen- und Arylengruppen sind die vorstehend genannten Gruppen. Bevorzugt bedeutet $R^1$ unabhängig voneinander unsubstituiertes oder substituiertes Alkyl, unsubstituiertes oder substituiertes Aryl, unsubstituiertes oder substituiertes Heteroaryl, $SiR^3_3$, Halogen, bevorzugt F, $OR^3$, $SR^3$, $NR^3_2$, $CF_3$ oder CN. Ganz besonders bevorzugt bedeutet $R^1$ unabhängig voneinander unsubstituiertes oder substituiertes Alkyl, unsubstituiertes oder substituiertes Aryl, unsubstituiertes oder substituiertes Heteroaryl oder $SiR_3{}^3$. Weiter ganz besonders bevorzugt bedeutet $R^1$ Methyl, iso-Propyl und tert.-Butyl; unsubstituiertes oder substituiertes $C_6$-Aryl, wobei als Substituenten insbesondere Methyl oder Isopropyl geeignet sind, wobei ortho-disubstituierte $C_6$-Aryle besonders bevorzugt sind; oder $C_5$- oder $C_6$-Heteroaryl, z. B.

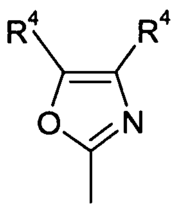 , 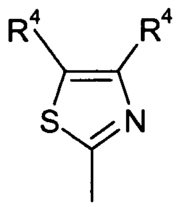 , 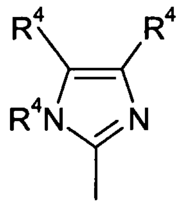 ,

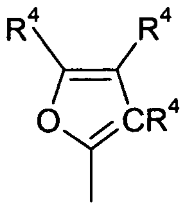 , 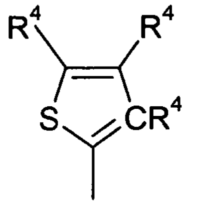 , 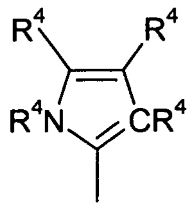

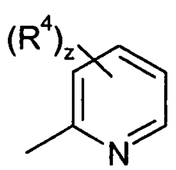 , 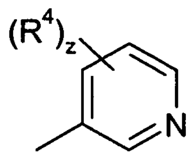 , 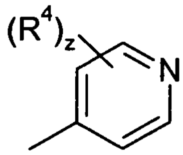

worin

$R^4$ unabhängig voneinander unsubstituiertes oder substituiertes Alkyl, unsubstituiertes oder substituiertes Aryl, unsubstituiertes oder substituiertes Herteroaryl oder $SiR^3_3$ bedeutet, bevorzugt Wasserstoff, Deuterium, Methyl, Ethyl, n-Propyl, isoPropyl, n-Butyl, sec-Butyl, iso-Butyl oder tert-Butyl; unsubstituiertes oder substituiertes $C_6$-Aryl oder $C_5$- oder $C_6$-Heteroaryl, besonders bevorzugt Wasserstoff; und

z 0, 1, 2, 3 oder 4, bevorzugt 0, 1 oder zwei;

bedeuten.

$R^2$ bedeutet unabhängig voneinander unsubstituiertes oder substituiertes Alkyl, unsubstituiertes oder substituiertes Aryl oder unsubstituiertes oder substituiertes Heteroaryl, oder zwei Reste $R^2$ oder ein Rest $R^2$ und ein Rest $R^1$ können gemeinsam eine gegebenenfalls substituierte Alkylen- oder Arylenbrücke bilden. Dabei können die beiden Reste $R^2$ bzw. $R^1$ und $R^2$ zu einem einzigen Zyklus der Liganden der allgemeinen Formel (I) oder zu zwei verschiedenen Zyklen des Liganden der allgemeinen Formel (I) gehören; wobei geeignete und bevorzugte Alkyl-, Aryl- und Heteroarylreste, geeignete Alkylen- oder Arylenbrücken sowie geeignete Substituenten vorstehend genannt sind. Bevorzugt ist $R^2$ Methyl, Ethyl, iso-Propyl, n-Propyl, n-Butyl, iso-Butyl, sec-Butyl oder tert.-Butyl oder $C_6$-Aryl, das unsubstituiert oder substituiert sein kann, bevorzugt Phenyl oder ortho,ortho-dialkylsubstituiertes Phenyl.

$R^3$ bedeutet unabhängig voneinander unsubstituiertes oder substituiertes Alkyl, unsubstituiertes oder substituiertes Aryl oder unsubstituiertes oder substituiertes Heteroaryl wobei geeignete und bevorzugte Alkyl-, Aryl- und Heteroarylreste sowie geeignete Substituenten vorstehend genannt sind. Bevorzugt ist $R^3$ Methyl, Ethyl, iso-Propyl, n-Propyl, n-Butyl, iso-Butyl, sec-Butyl oder tert.-Butyl oder $C_6$-Aryl, das unsubstituiert oder substituiert sein kann, bevorzugt Phenyl oder Tolyl.

Der erfindungsgemäße Metallkomplex enthält bevorzugt ein Metallatom ausgewählt aus der Gruppe bestehend aus Übergangsmetallen der Gruppe IB, IIB, IIIB, IVB, VB, VIB, VIIB, VIII des Periodensystems der Elemente (CAS-Version), in jeder für das entsprechende Metallatom möglichen Oxidationsstufe. Bevorzugt enthalten die erfindungsgemäßen Metallkomplexe ein Metallatom M ausgewählt aus der Gruppe bestehend aus Ir, Co, Rh, Ni, Pd, Pt, Fe, Ru, Os, Cr, Mo, W, Mn, Tc, Re, Ag, Au und Cu auf, besonders bevorzugt Ir, Os, Ru, Rh, Pd, Co, Ni und Pt, ganz besonders bevorzugt Ir, Pt, Rh, Ru und Os, in jeder für das entsprechende Metallatom möglichen Oxidationsstufe. Insbesondere bevorzugt werden Pt(II), Pt(IV), Ir(I), Ir(III), Os(II) und Ru(II), weiter insbesondere bevorzugt Pt(II), Ir(III) und Os(II) und ganz besonders bevorzugt Ir(III) eingesetzt.

Neben dem mindestens einen Liganden der allgemeinen Formeln (I) kann der erfindungsgemäße Metallkomplex weitere von den Liganden der allgemeinen Formel (I) verschiedene Liganden enthalten. Beispielsweise können neben mindestens einem Liganden der allgemeinen Formel (I) einer oder mehrere neutrale mono- oder bidentate Liganden K sowie gegebenenfalls einer oder mehrere mono- oder dianionische Liganden J, die mono- oder bidentat sein können, vorliegen. Des Weiteren können unterschiedliche Liganden der Formel (I) in dem erfindungsgemäßen Metallkomplex vorliegen. Dabei ist unter einem bidentaten Liganden ein Ligand zu verstehen, der an zwei Stellen an das Metallatom M koordiniert ist. Im Sinne der vorliegenden Anmeldung wird der Begriff "zweizähnig" synonym mit dem Begriff "bidentat" verwendet.

Unter einem monodentaten Liganden ist ein Ligand zu verstehen, der an einer Stelle des Liganden mit dem Metallatom M koordiniert.

Somit betrifft die vorliegende Erfindung in einer bevorzugten Ausführungsform Metallkomplexe der allgemeinen Formel (II)

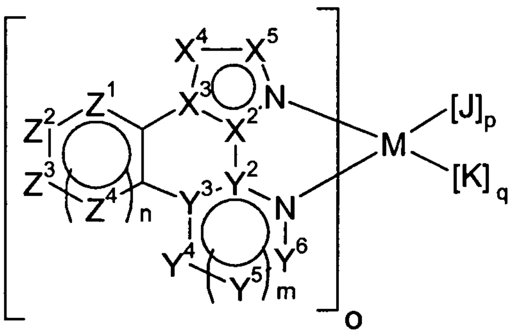

(II)

worin bedeuten:

M Metallatom ausgewählt aus der Gruppe bestehend aus Übergangsmetallen der Gruppe IB, IIB, IIIB, IVB, VB, VIB, VIIB, VIII des Periodensystems der Elemente (CAS-Version), in jeder für das entsprechende Metallatom möglichen Oxidationsstufe; bevorzugt Ir(III), Pt(II) oder Os(II), besonders bevorzugt Ir(III);

J mono- oder dianionischer Ligand, der mono- oder bidentat sein kann, bevorzugt bidentater, monoanionischer Ligand;

K neutraler, mono- oder bidentater Ligand, der im Allgemeinen nicht photoaktiv ist: bevorzugte Liganden K sind Phosphine, insbesondere Trialkylphosphine, z. B. $PEt_3$, $PnBu_3$, Triarylphosphine, z. B. $PPh_3$; Phosphonate und

Derivate davon, Arsenate und Derivate davon, Phosphite, CO, Nitrile, Amine, Diene, die einen π-Komplex mit M bilden können, z. B. 2,4-Hexadien, $\eta^4$-Cyclooctadien und $\eta^2$-Cyclooctadien (jeweils 1,3 und 1,5), Allyl, Methallyl, Cycloocten, Norbornadien und neutralen Biscarbene, z.B. die in WO 2008/000726 offenbarten neutralen Biscarbene;

o 1, 2, 3 oder 4; wobei o bevorzugt für M = Ir(III) 1, 2 oder 3, besonders bevorzugt 2 oder 3 und für M = Pt(II) oder Os(II) 1 oder 2 bedeutet;

p 0, 1, 2, 3 oder 4; wobei p bevorzugt für M = Ir(III) 0, 1 2, 3 oder 4, besonders bevorzugt 0 oder 2 und für M = Pt(II) und Os(II) 0, 1 oder 2, besonders bevorzugt 0 oder 2 für M = Pt(II) und besonders bevorzugt 0 für M = Os(II) bedeutet, wobei p die Zahl der Bindungsstellen zum Metall M bedeutet, d.h., wenn p = 2 ist, kann es sich um zwei monodentate oder einen bidentaten Liganden handeln;

q 0, 1, 2, 3 oder 4; wobei q bevorzugt für M = Ir(III) 0, 1 oder 2, besonders bevorzugt 0; für M = Pt(II) 0 oder 1, besonders bevorzugt 0, und für Os(II) 2 oder 3, besonders bevorzugt 2, bedeutet, wobei q die Zahl der Bindungsstellen zum Metall M bedeutet, d.h., wenn q = 2 ist, kann es sich um zwei monodentate oder einen bidentaten Liganden handeln;

wobei o, p und q von der Oxidationsstufe und Koordinationszahl des eingesetzten Metallatoms und der Ladung der Liganden abhängig sind.

Für den Fall, dass die Zahl o, p oder q > 1 ist, können die eingesetzten Liganden der Formel (I), K oder J jeweils gleich oder verschieden sein.

Für M = Ir(III) ist die Summe o, p + q in dem erfindungsgemäßen Metallkomplex der Formel (II) im Allgemeinen 3 oder 4 oder 5, d.h. in dem Fall, wenn 3 Liganden der Formel (I) vorliegen, ist o 3 und wenn 2 Liganden der Formel (I) und z.B. 1 bidentater, monoanionischer Ligand J vorliegt, ist o 2 und p 2, und in dem Fall, wenn z. B. 2 Liganden der Formel (I), 1 bidentater, monoanionischer Ligand J und 1 neutraler monoanionischer Ligand K vorliegen, ist o 2, p 2 und q 1. Für M = Pt(II) ist die Summe o + p in dem erfindungsgemäßen Metallkomplex der Formel (II) im Allgemeinen 2 oder 3, d.h. in dem Fall, wenn 2 Liganden der Formel (I) vorliegen, ist o 2 und wenn 1 Ligand der Formel (I) und z.B. 1 bidentater, monoanionischer Ligand J vorliegt, ist o 1 und p 2, wobei o jeweils mindestens 1 ist. Für Os(II) ist die Summe o, p + q in dem erfindungsgemäßen Metallkomplex der Formel (II) im Allgemeinen 4 oder 5, d.h. wenn 2 Liganden der Formel (I) und z.B. 1 bidentater, neutraler Ligand K vorliegt, ist o 2 und q 2, und in dem Fall, wenn z. B. 1 Ligand der Formel (I), 1 bidentater, monoanionischer Ligand J und 1 neutraler bidentater Ligand K vorliegen, ist o 1, p 2 und q 2.

Die Symbole $X^2$, $X^3$, $X^4$, $X^5$, $Y^2$, $Y^3$, $Y^4$, $Y^5$, $Y^6$, $Z^1$, $Z^2$, $Z^3$, $Z^4$, m und n in dem Metallkomplex der Formel (II) weisen die vorstehend genannten Bedeutungen auf.

Falls verschiedene Isomere der erfindungsgemäßen Metallkomplexe vorliegen können, umfasst die vorliegende Erfindung sowohl jeweils die einzelnen Isomere der Metallkomplexe, als auch Gemische verschiedener Isomere in jedem beliebigen Mischungsverhältnis. Im Allgemeinen können verschiedene Isomere der Metallkomplexe nach dem Fachmann bekannten Verfahren, z. B. durch Chromatographie, Sublimation oder Kristallisation, getrennt werden.

Üblicherweise werden als bidentate, monoanionische Liganden J nicht photoaktive oder photoaktive (z.B. heteroleptische Komplexe mit Carbenen, Phenylpyridinen oder Phenylimidazolen) Liganden eingesetzt. Geeignete Liganden J sind z.B. bidentate, monoanionische Liganden der allgemeinen Formel

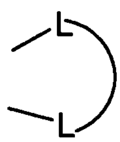

, worin L jeweils unabhängig voneinander ausgewählt ist aus O, N und C. Bevorzugt sind bidentate, monoanionische Liganden J, worin beide Gruppen L O, C oder N bedeuten, oder eine Gruppe L O bedeutet und die andere Gruppe L N oder C bedeutet, oder eine Gruppe L C bedeutet und die anderen Gruppe L N bedeutet. Besonders bevorzugte bidentate, monoanionische Liganden sind Acetylacetonat und dessen Derivate, Picolinat und dessen Derivate, bidentate, monoanionische Carbenliganden und dessen Derivate, z.B. Carbenliganden, die in WO 2005/019373, WO 2005/0113704, WO 2006/018292, WO 2006/056418, WO 2007/115981, WO 2007/115970, WO 2008/000727, WO 2006/067074, WO 2006/106842, WO 2007/018067, WO 2007/058255, WO 2007/069542, US 2007/108891, WO 2007/058080, WO 2007/058104 genannt sind, sowie die in WO 02/15645, WO 2005/123873, US 2007/196690, WO 2006/121811 genannten bidentaten, monoanionischen Liganden. Besonders bevorzugt sind die bidentaten, monoanionischen Liganden ausgewählt aus der Gruppe bestehend aus Acetylacetonat, Picolinat, Carbenen wie N-Methyl-N-arylimidazolcarben, Arylpyridinen wie 2-Arylpyridinen, insbesondere Phenylpyridinen wie 2-Phenylpyridin, Arylimidazolen wie 2-Arylimidazolen, insbesondere Phenylimidazolen, wie 2-Phenylimidazol und Derivaten der vorstehend genannten Verbindungen.

In einer besonders bevorzugten Ausführungsform weist der erfindungsgemäße Metallkomplex die allgemeine Formel (IIa) auf.

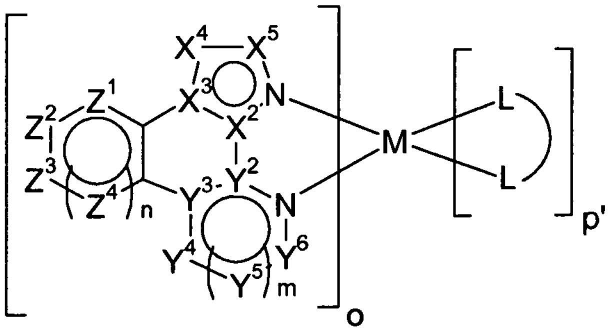

(IIa)

worin bedeuten

M Metallatom ausgewählt aus der Gruppe bestehend aus Übergangsmetallen der Gruppe IB, IIB, IIIB, IVB, VB, VIB, VIIB, VIII des Periodensystems der Elemente (CAS-Version), in jeder für das entsprechende Metallatom möglichen Oxidationsstufe; bevorzugt Ir(III) oder Pt(II), besonders bevorzugt Ir(III);

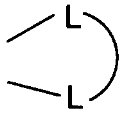

bidentater monoanionischer Ligand;

o 1, 2, 3, 4; wobei o bevorzugt für M = Ir(III) 1, 2 oder 3, besonders bevorzugt 2 oder 3 und für M = Pt(II) 1 oder 2; bedeutet

p' 0, 1 oder 2; wobei p' bevorzugt für M = Ir(III) 0, 1 oder 2, besonders bevorzugt 0 oder 1 und für M = Pt(II) 0 oder 1; bedeutet, wobei p' die Zahl der Liganden

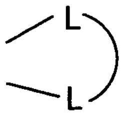

bedeutet;

wobei o und p' von der Oxidationsstufe und Koordinationszahl des eingesetzten Metallatoms abhängig sind.

Für M = Ir(III) ist die Summe o + p' in den erfindungsgemäßen Metallkomplexen der Formel (IIa) besonders bevorzugt 3, und für M = Pt(II) ist die Summe o + p' besonders bevorzugt 2, wobei o jeweils mindestens 1 ist.

Die weiteren Symbole und Indizes in dem Metallkomplex der Formel (IIa) weisen die vorstehend genannten Bedeutungen auf. Des Weiteren sind weitere Ausführungsformen von M, des bidentaten, monoanionischen Liganden sowie von o und p' die vorstehend für M, den bidentaten, monoanionischen Liganden, o und p' (bzw. p, wobei p' = 1 p = 2 entspricht) genannten Ausführungsformen.

In einer besonders bevorzugten Ausführungsform betrifft die vorliegende Erfindung Metallkomplexe der Formel (IIaa)

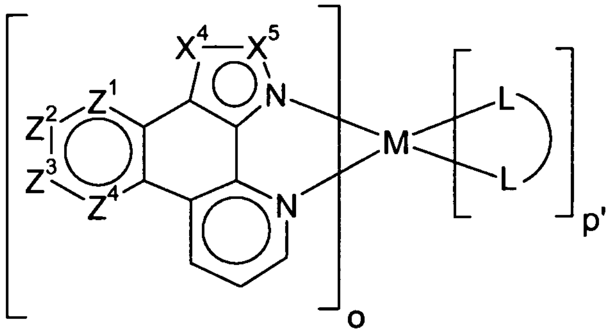

(IIaa)

worin M, o und p' in Formel (IIaa) unabhängig voneinander die vorstehend genannten Bedeutungen aufweisen; und in den Verbindungen der Formel IIaa:

| | |
|---|---|
| $X^4$, $X^5$ | unabhängig voneinander $CR^1$, CH oder N; |
| $Z^1$, $Z^2$, $Z^3$, $Z^4$ | CH oder N; und |

bedeuten;
wobei $R^1$ die vorstehend genannten Bedeutungen aufweist.

Im Folgenden sind beispielhaft bevorzugte erfindungsgemäße Metallkomplexe der Formel (IIaa) aufgeführt.

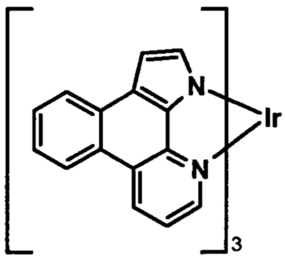

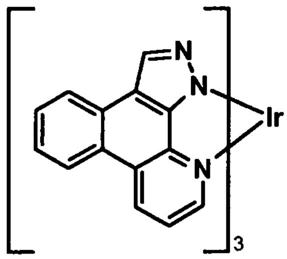

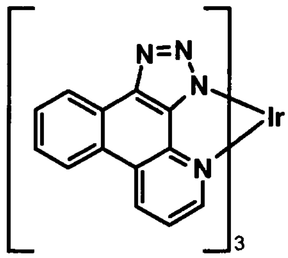

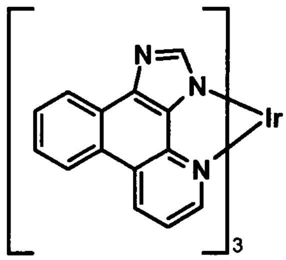

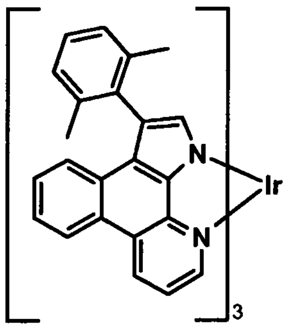

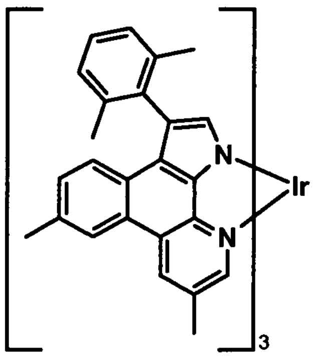

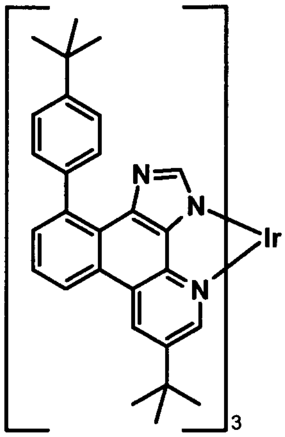
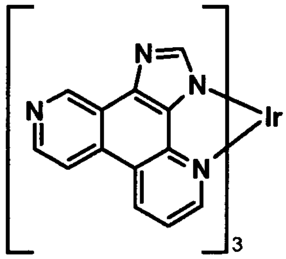
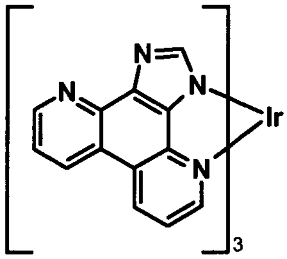

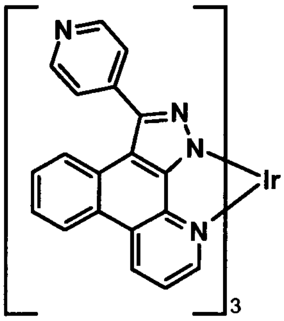
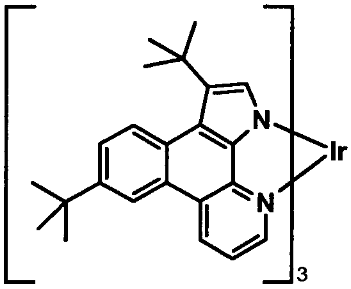
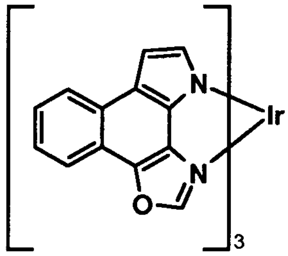

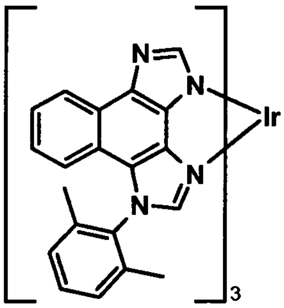
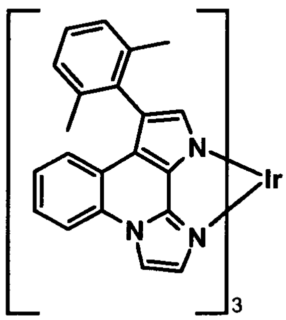
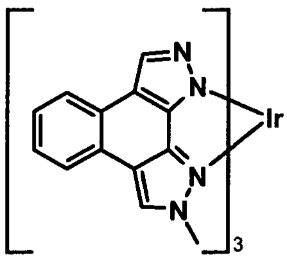

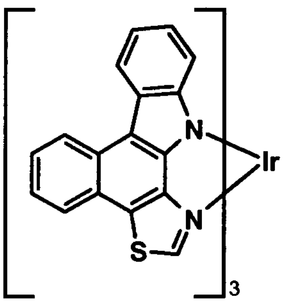
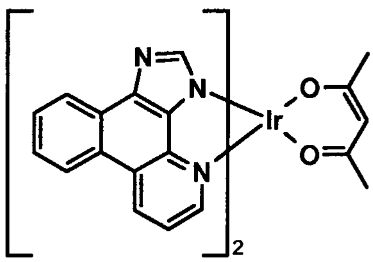
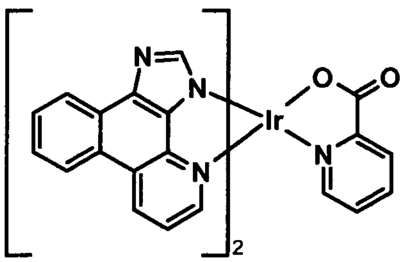

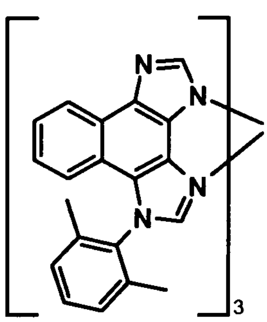

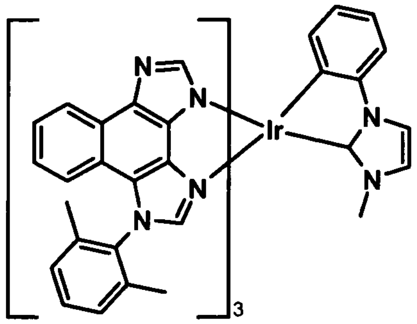

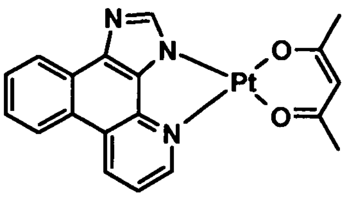

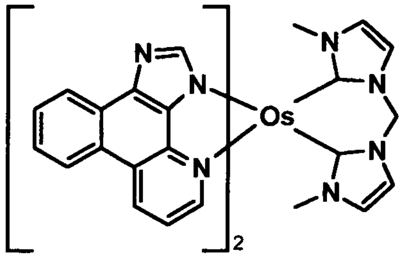

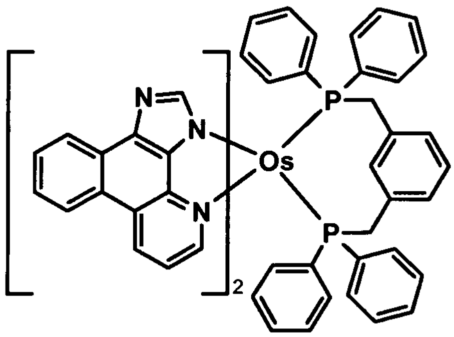

Die erfindungsgemäßen Metallkomplexe können gemäß dem Fachmann bekannten Verfahren bzw. analog zu dem Fachmann bekannten Verfahren hergestellt werden. Geeignete Herstellungsverfahren sind z. B. analog zu den in den Beispielen von WO 2007/095118 genannten Verfahren.

Üblicherweise werden die erfindungsgemäßen Metallkomplexe ausgehend von den den Liganden der allgemeinen Formel (I) entsprechenden Ligandvorläufem hergestellt. Dabei erfolgt die Herstellung der erfindungsgemäßen Metallkomplexe durch Umsetzung von mindestens einem Ligandvorläufer basierend auf den Liganden der allgemeinen Formel (I) mit einem Metallkomplex enthaltend mindestens ein Metall M, wobei M die vorstehend genannten Bedeutungen aufweist.

Das molare Verhältnis zwischen den Ligandvorläufern basierend auf den Liganden der Formel (I) und dem Metallkomplex enthaltend mindestens ein Metall M ist von der Struktur des gewünschten erfindungsgemäßen Metallkomplexes abhängig sowie von der Anzahl der Liganden der Formel (I). In dem Fall, dass o in den erfindungsgemäßen Metallkomplexen > 1 ist, ist es möglich, dass diese Metallkomplexe durch Umsetzung des Metallkomplexes enthaltend mindestens ein Metall M mit identischen Ligandvorläufern oder durch Umsetzung mit verschiedenen Ligandvorläufern erhalten werden. Geeignete Verfahren und Umsetzungsreihenfolgen zur Herstellung der verschiedenen erfindungsgemäßen Metallkomplexe sind dem Fachmann bekannt.

Bei dem mit dem Ligandvorläufer umzusetzenden Metallkomplex enthaltend mindestens ein Metall M handelt es sich um einen Metallkomplex enthaltend mindestens ein Metallatom ausgewählt aus der Gruppe bestehend aus Übergangsmetallen der Gruppe IB, IIB, IIIB, IVB, VB, VIB, VIIB, VIII des Periodensystems der Elemente (CAS-Version), bevorzugt ausgewählt aus der Gruppe bestehend aus Ir, Co, Rh, Ni, Pd, Pt, Fe, Ru, Os, Cr, Mo, W, Mn, Tc, Re und Cu, besonders bevorzugt Ir, Os, Ru, Rh, Pd, Co und Pt, ganz besonders bevorzugt Ir, Pt, Rh, Pd, Ru und Os in jeder geeigneten für das entsprechende Metall möglichen Oxidationsstufe.

Geeignete mit dem Ligandvorläufer umzusetzende Metallkomplexe sind dem Fachmann bekannt. Beispiele für geeignete Metallkomplexe sind: $Pd(OAc)_2$, $Pt(cod)Cl_2$, $Pt(cod)Me_2$, $Pt(acac)_2$, $Pt(PPh_3)_2Cl_2$, $PtCl_2$, $[Rh(cod)Cl]_2$, $Rh(acac)CO(PPh_3)$, $Rh(acac)(CO)_2$, $Rh(cod)_2BF_4$, $RhCl(PPh_3)_3$, $RhCl_3$ x $nH_2O$, $Rh(acac)_3$, $[Os(CO)_3I_2]_2$, $[Os_3(CO)_{12}]$,

$OsH_4$ $(PPh_3)_3Cp_2Os$, $Cp^*_2Os$, $H_2OsCl_6$ x $6H_2O$, $OsCl_3$ x $H_2O$, $Ru(acac)_3$, $RuCl_2(cod)$, Ru(2-methylallyl)$_2$(cod), [(μ-Cl)Ir($\eta^4$-1,5-cod)]$_2$, [(μ-Cl)Ir($\eta^2$-coe)$_2$]$_2$, $Ir(acac)_3$, $IrCl_3$ x $nH_2O$, $(tht)_3IrCl_3$, Ir($\eta^3$-allyl)$_3$, Ir($\eta^3$-metallyl)$_3$, worin cod Cyclooctadien, coe Cycloocten, acac Acetylacetonat und tht Tetrahydrothiophen bedeuten. Die Metallkomplexe können gemäß dem Fachmann bekannten Verfahren hergestellt werden bzw. sind kommerziell erhältlich.

Im Anschluss an die vorstehend erwähnte Umsetzung des mit dem Ligandvorläufer umzusetzenden Metallkomplexes mit einem oder mehreren Ligandvorläufern wird der erhaltene erfindungsgemäße Metallkomplex im Allgemeinen nach dem Fachmann bekannten Verfahren aufgearbeitet und gegebenenfalls gereinigt. Üblicherweise erfolgen Aufarbeitung und Reinigung durch Extraktion, Säulenchromatographie und/oder Umkristallisation gemäß dem Fachmann bekannten Verfahren.

Die erfindungsgemäßen Metallkomplexe werden in organischen Leuchtdioden (OLEDs) verwendet. Sie eignen sich als Emittersubstanzen, da sie eine Emission (Elektrolumineszenz) im sichtbaren Bereich des elektromagnetischen Spektrums aufweisen. Mithilfe der erfindungsgemäßen Metallkomplexe als Emittersubstanzen ist es möglich, Verbindungen bereitzustellen, die Elektrolumineszenz, bevorzugt Elektrophosphoreszenz, insbesondere bei Wellenlängen von 450 bis 500 nm, des elektromagnetischen Spektrums mit guter Effizienz zeigen. Dabei ist die Quantenausbeute hoch und insbesondere die Lebensdauer und die Stabilität der erfindungsgemäßen Metallkomplexe im Device hoch.

Des Weiteren sind die erfindungsgemäßen Metallkomplexe als Elektronen-, Excitonenoder Lochblocker, Lochleiter, Elektronenleiter, Lochinjektionsschicht oder Matrixmaterial in OLEDs geeignet, in Abhängigkeit von den eingesetzten Liganden und dem eingesetzten Zentralmetall.

Organische Leuchtdioden (OLEDs) sind grundsätzlich aus mehreren Schichten aufgebaut:

1. Anode (1)
2. Löcher-transportierende Schicht (2)
3. Licht-emittierende Schicht (3)
4. Elektronen-transportierende Schicht (4)
5. Kathode (5)

Es ist jedoch auch möglich, dass die OLED nicht alle der genannten Schichten aufweist, zum Beispiel ist eine OLED mit den Schichten (1) (Anode), (3) (Licht-emittierende Schicht) und (5) (Kathode) ebenfalls geeignet, wobei die Funktionen der Schichten (2) (Löcher-transportierende Schicht) und (4) (Elektronen-transportierende Schicht) durch die angrenzenden Schichten übernommen werden. OLEDs, die die Schichten (1), (2), (3) und (5) bzw. die Schichten (1), (3), (4) und (5) aufweisen, sind ebenfalls geeignet.

Die erfindungsgemäßen Metallkomplexe können in verschiedenen Schichten einer OLED eingesetzt werden. Ein weiterer Gegenstand der vorliegenden Erfindung ist daher eine OLED enthaltend mindestens einen erfindungsgemäßen Metallkomplex sowie die Verwendung mindestens eines erfindungsgemäßen Metallkomplexes in OLEDs. Die erfindungsgemäßen Metallkomplexe werden bevorzugt in der Licht-emittierenden Schicht, besonders bevorzugt als Emittermoleküle, eingesetzt. Ein weiterer Gegenstand der vorliegenden Erfindung ist daher eine Licht-emittierende Schicht enthaltend mindestens einen erfindungsgemäßen Metallkomplex als Matrixmaterial oder Emittermolekül, bevorzugt als Emittermolekül. Bevorzugte erfindungsgemäße Metallkomplexe sind vorstehend genannt.

Die erfindungsgemäßen Metallkomplexe können in Substanz - ohne weitere Zusätze - in der Licht-emittierenden Schicht oder einer anderen Schicht der OLED, bevorzugt in der Licht-emittierenden Schicht, vorliegen. Es ist jedoch ebenfalls möglich und bevorzugt, dass neben den erfindungsgemäßen Metallkomplexen weitere Verbindungen in den Schichten, bevorzugt in der Licht-emittierenden Schicht, vorliegen. Beispielsweise kann in der Licht-emittierenden Schicht ein fluoreszierender Farbstoff anwesend sein, um die Emissionsfarbe des als Emittermoleküls eingesetzten erfindungsgemäßen Metallkomplexes zu verändern. Des Weiteren kann - in einer bevorzugten Ausführungsform - mindestens ein Matrixmaterial eingesetzt werden. Geeignete Matrixmaterialien sind dem Fachmann bekannt. Im Allgemeinen wird das Matrixmaterial so ausgewählt, dass die Bandlücke des Matrixmaterials größer ist als die Bandlücke des als Emitter eingesetzten, erfindungsgemäßen Metallkomplexes. Unter Bandlücke ist im Sinne der vorliegenden Anmeldung die Triplettenergie zu verstehen. Bevorzugt eingesetzte geeignete Matrixmaterialien, insbesondere bei Verwendung von erfindungsgemäßen Metallkomplexen als Emittermaterialien, die im blauen Bereich des elektromagnetischen Spektrums Licht emittieren, sind z. B. Carbenkomplexe, insbesondere die in WO 2005/019373, WO 2005/0113704, WO 2006/018292, WO 2006/056418, WO 2007/115981, WO 2008/000726 und WO 2008/000727 genannten Carbenkomplexe; Disilylcarbazole, z.B. 9-(4-tert.-Butyl-phenyl)-3,6-bis(triphenylsilylcarbazol), 9-(Phenyl)-3,6-bis(triphenylsilyl)carbazol sowie in der nicht vorveröffentlichten PCT-Anmeldung mit dem Aktenzeichen PCT/EP 2007/059648 genannte Disilylcarbazole und die in WO 2004/095889, EP 1617710, EP 1617711, WO 2006/112265, WO 2006/130598 aufgeführten Verbindungen.

Die einzelnen der vorstehend genannten Schichten der OLED können wiederum aus 2 oder mehreren Schichten aufgebaut sein. Beispielsweise kann die Löcher-transportierende Schicht aus einer Schicht aufgebaut sein, in die aus der Elektrode Löcher injiziert werden und einer Schicht, die die Löcher von der Lochinjektionsschicht weg in die Licht-emittierende Schicht transportiert. Die Elektronen-transportierende Schicht kann ebenfalls aus mehreren Schichten bestehen, zum Beispiel einer Schicht, worin Elektronen durch die Elektrode injiziert werden, und einer Schicht, die aus der Elektroneninjektionsschicht Elektronen erhält und in die Licht-emittierende Schicht transportiert. Diese genannten Schichten werden jeweils nach Faktoren wie Energieniveau, Temperaturresistenz und Ladungsträgerbeweglichkeit, sowie Energiedifferenz der genannten Schichten mit den organischen Schichten oder den Metallelektroden ausgewählt. Der Fachmann ist in der Lage, den Aufbau der OLEDs so zu wählen, dass er optimal an die erfindungsgemäßen, bevorzugt als Emittersubstanzen verwendeten Metallkomplexe angepasst ist.

Um besonders effiziente OLEDs zu erhalten, sollte das HOMO (höchstes besetztes Molekülorbital) der Lochtransportierenden Schicht mit der Arbeitsfunktion der Anode angeglichen sein und das LUMO (niedrigstes unbesetztes Molekülorbital) der elektronentransportierenden Schicht sollte mit der Arbeitsfunktion der Kathode angeglichen sein.

Ein weiterer Gegenstand der vorliegenden Anmeldung ist eine OLED enthaltend mindestens eine erfindungsgemäße Licht-emittierende Schicht. Die weiteren Schichten in dem OLED können aus einem beliebigen Material aufgebaut sein, das üblicherweise in solchen Schichten eingesetzt wird und dem Fachmann bekannt ist.

Geeignete Materialien für die vorstehend genannten Schichten (Anode, Kathode, Lochund Elektroneninjektionsmaterialien, Loch- und Elektronentransportmaterialien und Loch- und Elektronenblockermaterialien, Matrixmaterialien, Fluoreszenz- und Phosphoreszenzemitter) sind dem Fachmann bekannt und z.B. in H. Meng, N. Herron, Organic Small Molecule Materials for Organic Light-Emitting Devices in Organic Light-Emitting Materials and Devices, Ed.: Z. Li, H. Meng, Taylor & Francis, 2007, Chapter 3, Seiten 295 bis 411 genannt.

Die Anode (1) ist eine Elektrode, die positive Ladungsträger bereitstellt. Sie kann zum Beispiel aus Materialien aufgebaut sein, die ein Metall, eine Mischung verschiedener Metalle, eine Metalllegierung, ein Metalloxid oder eine Mischung verschiedener Metalloxide enthält. Alternativ kann die Anode ein leitendes Polymer sein. Geeignete Metalle umfassen die Metalle der Gruppen 11, 4, 5 und 6 des Periodensystems der Elemente sowie die Übergangsmetalle der Gruppen 8 bis 10. Wenn die Anode lichtdurchlässig sein soll, werden im Allgemeinen gemischte Metalloxide der Gruppen 12, 13 und 14 des Periodensystems der Elemente eingesetzt, zum Beispiel Indium-Zinn-Oxid (ITO). Es ist ebenfalls möglich, dass die Anode (1) ein organisches Material, zum Beispiel Polyanilin enthält, wie beispielsweise in Nature, Vol. 357, Seiten 477 bis 479 (11. Juni 1992) beschrieben ist. Zumindest entweder die Anode oder die Kathode sollten mindestens teilweise transparent sein, um das gebildete Licht auskoppeln zu können.

Geeignete Lochtransportmaterialien für die Schicht (2) der erfindungsgemäßen OLEDs sind zum Beispiel in Kirk-Othmer Encyclopedia of Chemical Technologie, 4. Auflage, Vol. 18, Seiten 837 bis 860, 1996 offenbart. Sowohl Löcher transportierende Moleküle als auch Polymere können als Lochtransportmaterial eingesetzt werden. Üblicherweise eingesetzte Löcher transportierende Moleküle sind ausgewählt aus der Gruppe bestehend aus 4,4'-Bis[N-(1-naphthyl)-N-phenyl-amino]biphenyl (α-NPD), N, N'-Diphenyl-N, N'-Bis(3-methylphenyl)-[1,1'-biphenyl]-4,4'-d i a m in (T PD), 1,1-Bis[(di-4-tolylamino)-phenyl]cyclohexan (TAPC), N, N'-Bis(4-methylphenyl)-N,N'-Bis(4-ethylphenyl)-[1,1'-(3,3'-dimethyl)biphenyl]-4,4'-diamin (ETPD), Tetrakis-(3-methylphenyl)-N,N,N',N'-2,5-phenylendiamin (PDA), α-Phenyl-4-N, N-diphenylaminostyrol (TPS), p-(Diethylamino)-benzaldehyddiphenylhydrazon (DEH), Triphenylamin (TPA), Bis[4-(N, N-diethylamino)-2-methylphenyl)(4-methyl-phenyl)methan (MPMP), 1-Phenyl-3-[p-(diethylamino)styryl]-5-[p-(diethylamino)phenyl]pyrazolin (PPR oder DEASP), 1,2-trans-Bis(9H-carbazol-9-yl)cyclobutan (DCZB), N,N,N',N'-tetrakis(4-methylphenyl)-(1,1'-biphenyl)-4,4'-diamin (TTB) 4,4',4"-tris(N,N-Diphenylamino)triphenylamin (TDTA) und Porphyrinverbindungen sowie Phthalocyaninen wie Kupferphthalocyanine. Üblicherweise eingesetzte Löcher transportierende Polymere sind ausgewählt aus der Gruppe bestehend aus Polyvinylcarbazolen, (Phenylmethyl)polysilanen, PEDOT (Poly(3,4-ethylendioxythiophen), bevorzugt PEDOT dotiert mit PSS (Polystyrolsulfonat), und Polyanilinen. Es ist ebenfalls möglich, Löcher transportierende Polymere durch Dotieren Löcher transportierender Moleküle in Polymere wie Polystyrol und Polycarbonat zu erhalten. Geeignete Löcher transportierende Moleküle sind die bereits vorstehend genannten Moleküle.

Geeignete Elektronentransportmaterialien für die Schicht (4) der erfindungsgemäßen OLEDs umfassen mit oxinoiden Verbindungen chelatisierte Metalle wie Tris(8-hydroxychinolato)aluminium ($Alq_3$), Verbindungen auf Phenanthrolinbasis wie 2,9-Dimethyl, 4,7-Diphenyl-1, 10-phenanthrolin (DDPA = BCP) oder 4,7-Diphenyl-1,10-phenanthrolin (DPA) und Azolverbindungen wie 2-(4-Biphenylyl)-5-(4-t-butylphenyl)-1,3,4-oxadiazol (PBD) und 3-(4-Biphenylyl)-4-phenyl-5-(4-t-butylphenyl)-1,2,4-triazol (TAZ). Dabei kann die Schicht (4) sowohl zur Erleichterung des Elektronentransports dienen als auch als Pufferschicht oder als Sperrschicht, um ein Quenchen des Excitons an den Grenzflächen der Schichten des OLEDs zu vermeiden. Vorzugsweise verbessert die Schicht (4) die Beweglichkeit der Elektronen und reduziert ein Quenchen des Excitons.

Von den vorstehend als Lochtransportmaterialien und Elektronen transportierende Materialien genannten Materialien können einige mehrere Funktionen erfüllen. Zum Beispiel sind einige der Elektronen leitenden Materialien gleichzeitig Löcher blockende Materialien, wenn sie ein tief liegendes HOMO aufweisen.

Die Ladungstransportschichten können auch elektronisch dotiert sein, um die Transporteigenschaften der eingesetzten Materialien zu verbessern, um einerseits die Schichtdicken großzügiger zu gestalten (Vermeidung von Pinholes/Kurzschlüssen) und um andererseits die Betriebsspannung des Devices zu minimieren. Beispielsweise können

die Lochtransportmaterialien mit Elektronenakzeptoren dotiert werden, zum Beispiel können Phthalocyanine bzw. Arylamine wie TPD oder TDTA mit Tetrafluorotetracyano-chinodimethan (F4-TCNQ) dotiert werden. Die Elektronentransportmaterialien können zum Beispiel mit Alkalimetallen dotiert werden, beispielsweise $Alq_3$ mit Lithium. Die elektronische Dotierung ist dem Fachmann bekannt und zum Beispiel in W. Gao, A. Kahn, J. Appl. Phys., Vol. 94, No. 1, 1 July 2003 (p-dotierte organische Schichten); A. G. Werner, F. Li, K. Harada, M. Pfeiffer, T. Fritz, K. Leo, Appl. Phys. Lett., Vol. 82, No. 25, 23 June 2003 und Pfeiffer et al., Organic Electronics 2003, 4, 89 - 103 offenbart.

Die Kathode (5) ist eine Elektrode, die zur Einführung von Elektronen oder negativen Ladungsträgern dient. Die Kathode kann jedes Metall oder Nichtmetall sein, das eine geringere Arbeitsfunktion aufweist als die Anode. Geeignete Materialien für die Kathode sind ausgewählt aus der Gruppe bestehend aus Alkalimetallen der Gruppe 1, zum Beispiel Li, Cs, Erdalkalimetallen der Gruppe 2, Metallen der Gruppe 12 des Periodensystems der Elemente, umfassend die Seltenerdmetalle und die Lanthanide und Aktinide. Des Weiteren können Metalle wie Aluminium, Indium, Calcium, Barium, Samarium und Magnesium sowie Kombinationen davon eingesetzt werden. Weiterhin können Lithium enthaltende organometallische Verbindungen oder LiF zwischen der organischen Schicht und der Kathode aufgebracht werden, um die Betriebsspannung (Operating Voltage) zu vermindern.

Die OLED gemäß der vorliegenden Erfindung kann zusätzlich weitere Schichten enthalten, die dem Fachmann bekannt sind. Beispielsweise kann zwischen der Schicht (2) und der Licht emittierenden Schicht (3) eine Schicht aufgebracht sein, die den Transport der positiven Ladung erleichtert und/oder die Bänderlücke der Schichten aneinander anpasst. Alternativ kann diese weitere Schicht als Schutzschicht dienen. In analoger Weise können zusätzliche Schichten zwischen der Licht emittierenden Schicht (3) und der Schicht (4) vorhanden sein, um den Transport der negativen Ladung zu erleichtern und/oder die Bänderlücke zwischen den Schichten aneinander anzupassen. Alternativ kann diese Schicht als Schutzschicht dienen.

In einer bevorzugten Ausführungsform enthält die erfindungsgemäße OLED zusätzlich zu den Schichten (1) bis (5) mindestens eine der im Folgenden genannten weiteren Schichten:

- eine Loch-Injektionsschicht zwischen der Anode (1) und der Löchertransportierenden Schicht (2);
- eine Blockschicht für Elektronen und/oder Excitonen zwischen der Löchertransportierenden Schicht (2) und der Licht-emittierenden Schicht (3);
- eine Blockschicht für Löcher und/oder Excitonen zwischen der Licht-emittierenden Schicht (3) und der Elektronen-transportierenden Schicht (4);
- eine Elektronen-Injektionsschicht zwischen der Elektronen-transportierenden Schicht (4) und der Kathode (5).

Wie bereits vorstehend erwähnt, ist es jedoch auch möglich, dass die OLED nicht alle der genannten Schichten (1) bis (5) aufweist, zum Beispiel ist eine OLED mit den Schichten (1) (Anode), (3) (Licht-emittierende Schicht) und (5) (Kathode) ebenfalls geeignet, wobei die Funktionen der Schichten (2) (Löcher-transportierende Schicht) und (4) (Elektronen-transportierende Schicht) durch die angrenzenden Schichten übernommen werden. OLEDs, die die Schichten (1), (2), (3) und (5) bzw. die Schichten (1), (3), (4) und (5) aufweisen, sind ebenfalls geeignet.

Dem Fachmann ist bekannt, wie er (zum Beispiel auf Basis von elektrochemischen Untersuchungen) geeignete Materialien auswählen muss. Geeignete Materialien für die einzelnen Schichten sowie geeignete OLED-Aufbauten sind dem Fachmann bekannt und z. B. in WO2005/113704 offenbart.

Des Weiteren kann jede der genannten Schichten der erfindungsgemäßen OLED aus zwei oder mehreren Schichten aufgebaut sein. Des Weiteren ist es möglich, dass einige oder alle der Schichten (1), (2), (3), (4) und (5) oberflächenbehandelt sind, um die Effizienz des Ladungsträgertransports zu erhöhen. Die Auswahl der Materialien für jede der genannten Schichten ist bevorzugt dadurch bestimmt, eine OLED mit einer hohen Effizienz zu erhalten.

Die Herstellung der erfindungsgemäßen OLED kann nach dem Fachmann bekannten Methoden erfolgen. Im Allgemeinen wird die OLED durch aufeinander folgende Dampfabscheidung (Vapor deposition) der einzelnen Schichten auf ein geeignetes Substrat hergestellt. Geeignete Substrate sind zum Beispiel Glas oder Polymerfilme. Zur Dampfabscheidung können übliche Techniken eingesetzt werden wie thermische Verdampfung, Chemical Vapor Deposition und andere. In einem alternativen Verfahren können die organischen Schichten aus Lösungen oder Dispersionen in geeigneten Lösungsmitteln beschichtet werden, wobei dem Fachmann bekannte Beschichtungstechniken angewendet werden. Zusammensetzungen, die neben dem mindestens einen erfindungsgemäßen Metallkomplex ein polymeres Material in einer der Schichten der OLED, bevorzugt in der Licht-emittierenden Schicht, aufweisen, werden im Allgemeinen mittels lösungsverarbeitenden Verfahren als Schicht aufgebracht.

Im Allgemeinen haben die verschiedenen Schichten folgende Dicken: Anode (1) 500 bis 5000 Å, bevorzugt 1000 bis 2000 Å; Löcher-transportierende Schicht (2) 50 bis 1000 Å, bevorzugt 200 bis 800 Å, Licht-emittierende Schicht (3) 10 bis 1000 Å, bevorzugt 100 bis 800 Å, Elektronen transportierende Schicht (4) 50 bis 1000 Å, bevorzugt 200 bis 800 Å, Kathode (5) 200 bis 10.000 Å, bevorzugt 300 bis 5000 Å. Die Lage der Rekombinationszone von Löchern und Elektronen in der erfindungsgemäßen OLED und somit das Emissionsspektrum der OLED können durch die relative Dicke jeder Schicht beeinflusst werden. Das bedeutet, die Dicke der Elektronentransportschicht sollte bevorzugt so

gewählt werden, dass die Elektronen/Löcher Rekombinationszone in der Licht-emittierenden Schicht liegt. Das Verhältnis der Schichtdicken der einzelnen Schichten in der OLED ist von den eingesetzten Materialien abhängig. Die Schichtdicken von gegebenenfalls eingesetzten zusätzlichen Schichten sind dem Fachmann bekannt.

Durch Einsatz der erfindungsgemäßen Metallkomplexe in mindestens einer Schicht der erfindungsgemäßen OLED, bevorzugt als Emittermolekül in der Licht-emittierenden Schicht der erfindungsgemäßen OLEDs, können OLEDs mit hoher Effizienz erhalten werden. Die Effizienz der erfindungsgemäßen OLEDs kann des Weiteren durch Optimierung der anderen Schichten verbessert werden. Beispielsweise können hoch effiziente Kathoden wie Ca, Ba oder LiF eingesetzt werden. Geformte Substrate und neue Löcher-transportierende Materialien, die eine Reduktion der Operationsspannung oder eine Erhöhung der Quanteneffizienz bewirken, sind ebenfalls in den erfindungsgemäßen OLEDs einsetzbar. Des Weiteren können zusätzliche Schichten in den OLEDs vorhanden sein, um die Energielevel der verschiedenen Schichten einzustellen und um Elektrolumineszenz zu erleichtern.

Die erfindungsgemäßen OLEDs können in allen Vorrichtungen eingesetzt werden, worin Elektrolumineszenz nützlich ist. Geeignete Vorrichtungen sind bevorzugt ausgewählt aus stationären und mobilen Bildschirmen. Stationäre Bildschirme sind z.B. Bildschirme von Computern, Fernsehern, Bildschirme in Druckern, Küchengeräten sowie Reklametafeln, Beleuchtungen und Hinweistafeln. Mobile Bildschirme sind z.B. Bildschirme in Handys, Laptops, Kameras, insbesondere Digitalkameras, Fahrzeugen sowie Zielanzeigen an Bussen und Bahnen.

Weiterhin können die erfindungsgemäßen Metallkomplexe in OLEDs mit inverser Struktur eingesetzt werden. Bevorzugt werden die erfindungsgemäßen Metallkomplexe in diesen inversen OLEDs wiederum in der Licht-emittierenden Schicht eingesetzt. Der Aufbau von inversen OLEDs und die üblicherweise darin eingesetzten Materialien sind dem Fachmann bekannt.

**Patentansprüche**

1. Metallkomplex enthaltend mindestens einen Liganden der allgemeinen Formel (I)

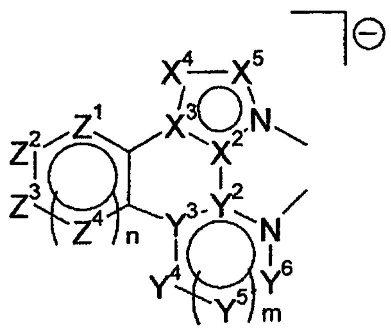

(I)

worin die Symbole die folgenden Bedeutungen aufweisen:

$Y^5$, $Z^4$ unabhängig voneinander $CR^1$, CH oder N;
$X^2$, $X^3$, $Y^2$, $Y^3$ unabhängig voneinander N oder C;
$X^4$, $X^5$ unabhängig voneinander $CR^1$, CH oder N;
$Y^4$, $Y^6$ unabhängig voneinander $CR^1$, CH oder N wobei $Y^4$ oder $Y^6$ im Falle von m = 0 des Weiteren $NR^1$, S oder O bedeuten können;
$Z^1$, $Z^2$, $Z^3$ unabhängig voneinander $CR^1$, CH oder N; wobei $Z^1$, $Z^2$ oder $Z^3$ im Falle von n = 0 des Weiteren $NR^1$, S oder O bedeuten können;
$R^1$ unabhängig voneinander unsubstituiertes oder substituiertes Alkyl, unsubstituiertes oder substituiertes Cycloalkyl, unsubstituiertes oder substituiertes Heterocycloalkyl, unsubstituiertes oder substituiertes Aryl, unsubstituiertes oder substituiertes Heteroaryl, unsubstituiertes oder substituiertes Alkenyl, unsubstituiertes oder substituiertes Cycloalkenyl, unsubstituiertes oder substituiertes Alkinyl, $SiR^2_3$, Halogen, ein Substituent mit Donor- oder Akzeptorwirkung ausgewählt aus Alkoxyresten oder Aryloxyresten, Esterresten, Aminogruppen, Amidresten, $CH_2F$-Gruppen, $CHF_2$-Gruppen, $CF_3$-Gruppen, CN-Gruppen, Thiogruppen, Sulfonsäuregruppen, Sulfonsäureestergruppen, Boronsäuregruppen, Boronsäureestergruppen, Phosphonsäuregruppen, Phosphonsäureestergruppen, Phosphinresten, Sulfoxidresten, Sulfonylresten, Sulfidresten, $SR^3$, Nitrogruppen, OCN, Boranresten, Stannatresten, Iminogruppen, Hydrazinresten, Hydrazonresten, Oximresten, Nitrosogruppen, Diazogruppen, Phosphinoxidgruppen, Hydroxygruppen oder SCN-Gruppen, des Weiteren können zwei Reste $R^1$ gemeinsam eine Alkylen- oder Arylenbrücke bilden;
$R^2$ unabhängig voneinander unsubstituiertes oder substituiertes Alkyl, unsubstituiertes oder substituiertes Aryl oder unsubstituiertes oder substituiertes Heteroaryl;
m, n unabhängig voneinander 0 oder 1, wobei die Gruppen $Z^4$ bzw. $Y^5$ bei n bzw. m = 0 nicht vorhanden sind.

**2.** Metallkomplex nach Anspruch 1, **dadurch gekennzeichnet, dass** m = 1 ist.

**3.** Metallkomplex nach Anspruch 1 oder 2, **dadurch gekennzeichnet, dass** n = 1 ist.

**4.** Metallkomplex nach einem der Ansprüche 1 bis 3, **dadurch gekennzeichnet, dass**

$X^4$, $X^5$, $Y^5$, $Y^6$, $Z^1$, $Z^2$, $Z^3$, $Z^4$ unabhängig voneinander $CR^1$, CH oder N;
$Y^4$ $CR^1$, CH oder N; wobei $Y^4$ im Falle von m = 0 des Weiteren $NR^1$, S oder O bedeuten kann;
$X^3$, $Y^3$ N oder C, bevorzugt C;
$X^2$, $Y^2$ N oder C, bevorzugt C;

bedeuten.

**5.** Metallkomplex nach Anspruch 3 oder 4, **dadurch gekennzeichnet, dass**

$Z^1$, $Z^2$, $Z^3$, $Z^4$ unabhängig voneinander $CR^1$, CH oder N bedeuten, wobei bevorzugt 0, 1 oder 2 Gruppen $Z^1$, $Z^2$, $Z^3$, $Z^4$ N bedeuten.

**6.** Metallkomplex nach einem der Ansprüche 2 bis 4, **dadurch gekennzeichnet, dass**

$Y^4$, $Y^5$, $Y^6$ unabhängig voneinander CH oder $CR^1$; und
$Y^2$, $Y^3$ C

bedeuten.

**7.** Metallkomplex nach einem der Ansprüche 1 bis 6, **dadurch gekennzeichnet, dass** der Metallkomplex die allgemeine Formel (II) aufweist

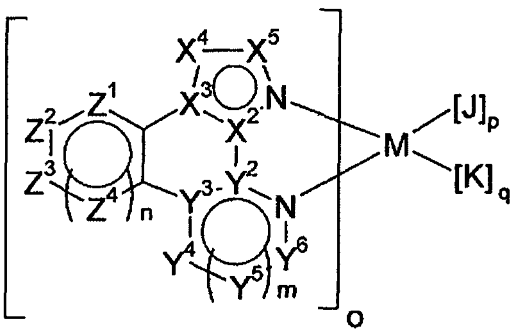

(II)

worin bedeuten:

M Metallatom ausgewählt aus der Gruppe bestehend aus Übergangsmetallen der Gruppe IB, IIB, IIIB, IVB, VB, VIB, VIIB, VIII des Periodensystems der Elemente (CAS-Version), in jeder für das entsprechende Metallatom möglichen Oxidationsstufe; bevorzugt Ir(III), Pt(II) oder Os(II), besonders bevorzugt Ir(III);
J mono- oder dianionischer Ligand, der mono- oder bidentat sein kann, bevorzugt bidentater, monoanionischer Ligand;
K neutraler, mono- oder bidentater Ligand, der im Allgemeinen nicht photoaktiv ist: bevorzugte Liganden K sind Phosphine, insbesondere Trialkylphosphine, z. B. $PEt_3$, $PnBu_3$, Triarylphosphine, z. B. $PPh_3$; Phosphonate und Derivate davon, Arsenate und Derivate davon, Phosphite, CO, Nitrile, Amine, Diene, die einen π-Komplex mit

M bilden können, z. B. 2,4-Hexadien, $\eta^4$-Cyclooctadien und $\eta^2$-Cyclooctadien (jeweils 1,3 und 1,5), Allyl, Methallyl, Cycloocten, Norbornadien und neutrale Biscarbene,

o 1, 2, 3 oder 4; wobei o bevorzugt für M = Ir(III) 1, 2 oder 3, besonders bevorzugt 2 oder 3 und für M = Pt(II) oder Os(II) 1 oder 2 bedeutet;

p 0, 1, 2, 3 oder 4; wobei p bevorzugt für M = Ir(III) 0, 1 2, 3 oder 4, besonders bevorzugt 0 oder 2 und für M = Pt(II) und Os(II) 0, 1 oder 2, besonders bevorzugt 0 oder 2 für M = Pt(II) und besonders bevorzugt 0 für M = Os(II) bedeutet, wobei p die Zahl der Bindungsstellen zum Metall M bedeutet, d.h., wenn p = 2 ist, kann es sich um zwei monodentate oder einen bidentaten Liganden handeln;

q 0, 1, 2, 3 oder 4; wobei q bevorzugt für M = Ir(III) 0, 1 oder 2, besonders bevorzugt 0; für M = Pt(II) 0 oder 1, besonders bevorzugt 0, und für Os(II) 2 oder 3, besonders bevorzugt 2, bedeutet, wobei q die Zahl der Bindungsstellen zum Metall M bedeutet, d.h., wenn q = 2 ist, kann es sich um zwei monodentate oder einen bidentaten Liganden handeln;

wobei o, p und q von der Oxidationsstufe und Koordinationszahl des eingesetzten Metallatoms und der Ladung der Liganden abhängig sind.

**8.** Metallkomplex nach Anspruch 7, **dadurch gekennzeichnet, dass** der Metallkomplex die allgemeine Formel (IIa) aufweist

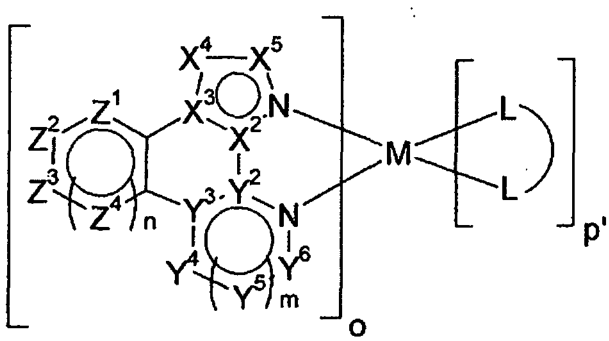

(IIa)

worin bedeuten

M Metallatom ausgewählt aus der Gruppe bestehend aus Übergangsmetallen der Gruppe IB, IIB, IIIB, IVB, VB, VIB, VIIB, VIII des Periodensystems der Elemente (CAS-Version), in jeder für das entsprechende Metallatom möglichen Oxidationsstufe; bevorzugt Ir(III) oder Pt(II), besonders bevorzugt Ir(III);

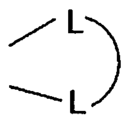

bidentater monoanionischer Ligand;

o 1, 2, 3, 4; wobei o bevorzugt für M = Ir(III) 1, 2 oder 3, besonders bevorzugt 2 oder 3 und für M = Pt(II) 1 oder 2;

p' 0, 1 oder 2; wobei p' bevorzugt für M = Ir(III) 0, 1 oder 2, besonders bevorzugt 0 oder 1 und für M = Pt{II) 0 oder 1; bedeutet, wobei p' die Zahl der Liganden

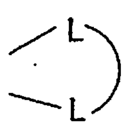

bedeutet;

wobei o und p' von der Oxidationsstufe und Koordinationszahl des eingesetzten Metallatoms abhängig sind.

**9.** Metallkomplex nach Anspruch 8, **dadurch gekennzeichnet, dass** L in dem bidentaten monoanionischen Liganden jeweils unabhängig voneinander ausgewählt ist aus O, N und C; bevorzugt sind monoanionische bidentate Liganden, worin beide Gruppen L O, C oder N bedeuten oder eine Gruppe L O bedeutet und die andere Gruppe L N oder C bedeutet oder eine Gruppe L C bedeutet und die andere Gruppe L N bedeutet; besonders bevorzugt sind die bidentaten monoanionischen Liganden ausgewählt aus der Gruppe bestehend aus Acetylacetonat, Picolinat, Carbenen, Arylpyridinen und Arylimidazolen und Derivaten der vorstehend genannten Verbindungen.

**10.** Metallkomplex nach einem der Ansprüche 8 bis 9, worin bedeuten:

M Ir(III) oder Pt(II), bevorzugt Ir(III);
o für M = Ir(III) 1, 2 oder 3, bevorzugt 2 oder 3; für M = Pt(II) 1 oder 2;
p' für M = Ir(III) 0, 1 oder 2, bevorzugt 0 oder 1; für M = Pt(II) 0 oder 1;

wobei die Summe o + p' für M = Ir(III) 3 und für M = Pt(II) 2 bedeutet und o mindestens 1 ist.

**11.** Organische Leuchtdiode enthaltend mindestens einen Metallkomplex enthaltend mindestens einen Liganden der allgemeinen Formel (I)

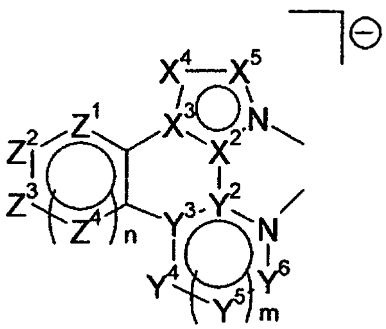

(I)

worin die Symbole die folgenden Bedeutungen aufweisen:

$Y^5$, $Z^4$ unabhängig voneinander $CR^1$, CH oder N;
$X^2$, $X^3$, $Y^2$, $Y^3$ unabhängig voneinander N oder C;
$X^4$, $X^5$ unabhängig voneinander $CR^1$, CH oder N;
$Y^4$, $Y^6$ unabhängig voneinander $CR^1$, CH oder N wobei $Y^4$ oder $Y^6$ im Falle von m = 0 des Weiteren $NR^1$, S oder O bedeuten können;
$Z^1$, $Z^2$, $Z^3$ unabhängig voneinander $CR^1$, CH oder N; wobei $Z^1$, $Z^2$ oder $Z^3$ im Falle von n = 0 des Weiteren $NR^1$, S oder O bedeuten können;
$R^1$ unabhängig voneinander unsubstituiertes oder substituiertes Alkyl, unsubstituiertes oder substituiertes Cycloalkyl, unsubstituiertes oder substituiertes Heterocycloalkyl, unsubstituiertes oder substituiertes Aryl, unsubstituiertes oder substituiertes Heteroaryl, unsubstituiertes oder substituiertes Alkenyl, unsubstituiertes oder substituiertes Cycloalkenyl, unsubstituiertes oder substituiertes Alkinyl, $SiR^2_3$, Halogen, ein Substituent mit Donor- oder Akzeptorwirkung; des Weiteren können zwei Reste $R^1$ gemeinsam eine Alkylen- oder Arylenbrücke bilden;
$R^2$ unabhängig voneinander unsubstituiertes oder substituiertes Alkyl, unsubstituiertes oder substituiertes Aryl oder unsubstituiertes oder substituiertes Heteroaryl;
m, n unabhängig voneinander 0 oder 1, wobei die Gruppen $Z^4$ bzw. $Y^5$ bei n bzw. m = 0 nicht vorhanden sind.

**12.** Licht-emittierende Schicht enthaltend mindestens einen Metallkomplex enthaltend mindestens einen Liganden der allgemeinen Formel (I)

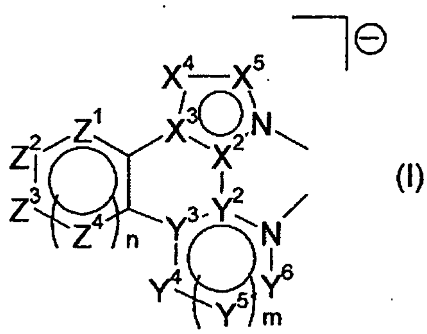

(I)

worin die Symbole die folgenden Bedeutungen aufweisen:

$Y^5$, $Z^4$ unabhängig voneinander $CR^1$, CH oder N;
$X^2$, $X^1$, $Y^2$. $Y^3$ unabhängig voneinander N oder C;
$X^4$, $X^5$ unabhängig voneinander $CR^1$, CH oder N;
$Y^4$, $Y^6$ unabhängig voneinander $CR^1$, CH oder N wobei $Y^4$ oder $Y^6$ im Falle von m = 0 des Weiteren $NR^1$, S oder O bedeuten können;
$Z^1$, $Z^2$, $Z^3$ unabhängig voneinander $CR^1$, CH oder N; wobei $Z^1$, $Z^2$ oder $Z^3$ im Falle von n = 0 des Weiteren $NR^1$, S oder O bedeuten können;
$R^1$ unabhängig voneinander unsubstituiertes oder substituiertes Alkyl, unsubstituiertes oder substituiertes Cycloalkyl, unsubstituiertes oder substituiertes Heterocycloalkyl, unsubstituiertes oder substituiertes Aryl, unsubstituiertes oder substituiertes Heteroaryl, unsubstituiertes oder substituiertes Alkenyl, unsubstituiertes oder substituiertes Cycloalkenyl, unsubstituiertes oder substituiertes Alkinyl, $SiR^2_3$, Halogen, ein Substituent mit Donor- oder Akzeptorwirkung; des Weiteren können zwei Reste $R^1$ gemeinsam eine Alkylen- oder Arylenbrücke bilden;
$R^2$ unabhängig voneinander unsubstituiertes oder substituiertes Alkyl, unsubstituiertes oder substituiertes Aryl oder unsubstituiertes oder substituiertes Heteroaryl;
m, n unabhängig voneinander 0 oder 1, wobei die Gruppen $Z^4$ bzw. $Y^5$ bei n bzw. m = 0 nicht vorhanden sind.

**13.** Organische Leuchtdiode enthaltend mindestens eine Licht-emittierende Schicht gemäß Anspruch 12.

**14.** Verwendung von mindestens einem Metallkomplex enthaltend mindestens einen Liganden der allgemeinen Formel (I)

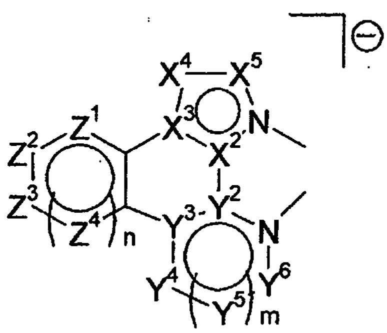

(I)

worin die Symbole die folgenden Bedeutungen aufweisen:

$Y^5$, $Z^4$ unabhängig voneinander $CR^1$, CH oder N;
$X^2$, $X^3$, $Y^2$, $Y^3$ unabhängig voneinander N oder C;
$X^4$, $X^5$ unabhängig voneinander $CR^1$, CH oder N;
$Y^4$, $Y^6$ unabhängig voneinander $CR^1$, CH oder N wobei $Y^4$ oder $Y^6$ im Falle von m = 0 des Weiteren $NR^1$, S oder O bedeuten können;
$Z^1$, $Z^2$, $Z^3$ unabhängig voneinander $CR^1$, CH oder N; wobei $Z^1$, $Z^2$ oder $Z^3$ im Falle von n = 0 des Weiteren

$NR^1$, S oder O bedeuten können;
$R^1$ unabhängig voneinander unsubstituiertes oder substituiertes Alkyl, unsubstituiertes oder substituiertes Cycloalkyl, unsubstituiertes oder substituiertes Heterocycloalkyl, unsubstituiertes oder substituiertes Aryl, unsubstituiertes oder substituiertes Heteroaryl, unsubstituiertes oder substituiertes Alkenyl, unsubstituiertes oder substituiertes Cycloalkenyl, unsubstituiertes oder substituiertes Alkinyl, $SiR^2_3$, Halogen, ein Substituent mit Donor- oder Akzeptorwirkung; des Weiteren können zwei Reste $R^1$ gemeinsam eine Alkylen- oder Arylenbrücke bilden;
$R^2$ unabhängig voneinander unsubstituiertes oder substituiertes Alkyl, unsubstituiertes oder substituiertes Aryl oder unsubstituiertes oder substituiertes Heteroaryl;
m, n unabhängig voneinander 0 oder 1, wobei die Gruppen $Z^4$ bzw. $Y^5$ bei n bzw. m = 0 nicht vorhanden sind,

in organischen Leuchtdioden.

**15.** Vorrichtung ausgewählt aus der Gruppe bestehend aus stationären Bildschirmen wie Bildschirmen von Computern, Fernsehern, Bildschirmen in Druckern, Küchengeräten sowie Reklametafeln, Beleuchtungen, Hinweistafeln und mobilen Bildschirmen wie Bildschirmen in Handys, Laptops, Digitalkameras, Fahrzeugen sowie Zielanzeigen an Bussen und Bahnen enthaltend mindestens eine organische Leuchtdiode gemäß Anspruch 11 oder 13.

## Claims

**1.** A metal complex comprising at least one ligand of the general formula (I)

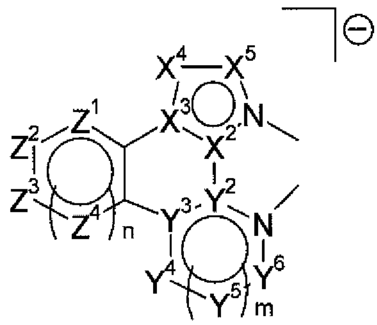

(I)

in which the symbols are each defined as follows:

$Y^5$, $Z^4$ are each independently $CR^1$, CH or N;
$X^2$, $X^3$, $Y^2$, $Y^3$ are each independently N or C;
$X^4$, $X^5$ are each independently $CR^1$, CH or N;
$Y^4$, $Y^6$ are each independently $CR^1$, CH or N, where $Y^4$ or $Y^6$, in the case that m = 0, may additionally be $NR^1$, S or 0;
$Z^1$, $Z^2$, $Z^3$ are each independently $CR^1$, CH or N; where $Z^1$, $Z^2$ or $Z^3$, in the case that n = 0, may additionally be $NR^1$, S or 0;
$R^1$ is independently unsubstituted or substituted alkyl, unsubstituted or substituted cycloalkyl, unsubstituted or substituted heterocycloalkyl, unsubstituted or substituted aryl, unsubstituted or substituted heteroaryl, unsubstituted or substituted alkenyl, unsubstituted or substituted cycloalkenyl, unsubstituted or substituted alkynyl, $SiR^2_3$, halogen, a substituent with donor or acceptor action selected from alkoxy radicals or aryloxy radicals, ester radicals, amino groups, amide radicals, $CH_2F$ groups, $CHF_2$ groups, $CF_3$ groups, CN groups, thio groups, sulfonic acid groups, sulfonic ester groups, boronic acid groups, boronic ester groups, phosphonic acid groups, phosphonic ester groups, phosphine radicals, sulfoxide radicals, sulfonyl radicals, sulfide radicals, $SR^3$, nitro groups, OCN, borane radicals, stannate radicals, imino groups, hydrazine radicals, hydrazone radicals, oxime radicals, nitroso groups, diazo groups, phosphine oxide groups, hydroxyl groups or SCN groups, in addition, two $R^1$ radicals together may form an alkylene or arylene bridge;
$R^2$ is independently unsubstituted or substituted alkyl, unsubstituted or substituted aryl or unsubstituted or substituted heteroaryl;
m, n are each independently 0 or 1, where the $Z^4$ and $Y^5$ groups are absent when, respectively, n and m = 0.

**2.** A metal complex according to claim 1, wherein m = 1.

**3.** A metal complex according to claim 1 or 2, wherein n = 1.

**4.** A metal complex according to any one of claims 1 to 3, wherein

$X^4$, $X^5$, $Y^5$, $Y^6$, $Z^1$, $Z^2$, $Z^3$, $Z^4$ are each independently $CR^1$, CH or N;
$Y^4$ is $CR^1$, CH or N; where $Y^4$, in the case that m = 0, may additionally be $NR^1$, S or O;
$X^3$, $Y^3$ are each N or C, preferably C;
$X^2$, $Y^2$ are each N or C, preferably C.

**5.** A metal complex according to claim 3 or 4, wherein

$Z^1$, $Z^2$, $Z^3$, $Z^4$ are each independently $CR^1$, CH or N, where preferably 0, 1 or 2 $Z^1$, $Z^2$, $Z^3$, $Z^4$ groups are each N.

**6.** A metal complex according to any one of claims 2 to 4, wherein

$Y^4$, $Y^5$, $Y^6$ are each independently CH or $CR^1$; and
$Y^2$, $Y^3$ are each C.

**7.** A metal complex according to any one of claims 1 to 6, wherein the metal complex has the general formula (II)

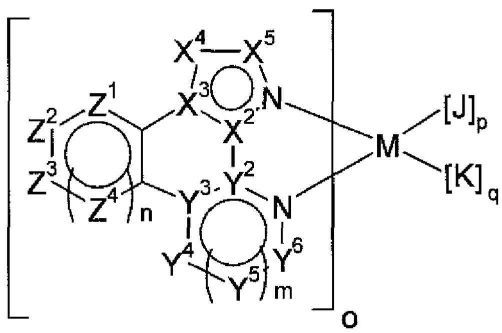

(II)

in which

M is a metal atom selected from the group consisting of transition metals of groups IB, IIB, IIIB, IVB, VB, VIB, VIIB, VIII of the Periodic Table of the Elements (CAS version), in any oxidation state possible for the particular metal atom; preferably Ir(III), Pt(II) or Os(II), more preferably Ir(III);
J is a mono- or dianionic ligand which may be mono- or bidentate, preferably a bidentate monoanionic ligand;
K is an uncharged, mono- or bidentate ligand which is generally not photoactive: preferred ligands K are phosphines, especially trialkylphosphines, e.g. $PEt_3$, $PnBu_3$, triarylphosphines, e.g. $PPh_3$; phosphonates and derivatives thereof, arsenates and derivatives thereof, phosphites, CO, nitriles, amines, dienes which can form a p-complex with M, for example 2,4-hexadiene, $\eta^4$-cyclooctadiene and $\eta^2$-cyclooctadiene (in each case 1,3 and 1,5), allyl, methallyl, cyclooctene, norbornadiene and uncharged biscarbenes,
o is 1, 2, 3 or 4; where o is preferably 1, 2 or 3 when M = Ir(III) more preferably 2 or 3, and is 1 or 2 when M = Pt (II) or Os (IT) ;
p is 0, 1, 2, 3 or 4; where p is preferably 0, 1, 2, 3 or 4 when M = Ir (III), more preferably 0 or 2, and is 0, 1 or 2 when M = Pt (II) and Os(II), more preferably 0 or 2 when M = Pt(II) and more preferably 0 when M = Os(II), where p is the number of bonding sites to the metal M, i.e., when p = 2, the ligands may be two monodentate ligands or one bidentate ligand;
q is 0, 1, 2, 3 or 4; where q is preferably 0, 1 or 2 when M = Ir(III), more preferably 0; is 0 or 1 when M = Pt(II), more preferably 0, and is 2 or 3 for Os(II), more preferably 2, where q is the number of bonding sites to the metal M, i.e., when q = 2, the ligands may be two monodentate ligands or one bidentate ligand;

where o, p and q depend on the oxidation state and coordination number of the metal atom used and on the charge

of the ligands.

**8.** A metal complex according to claim 7, wherein the metal complex has the general formula (IIa)

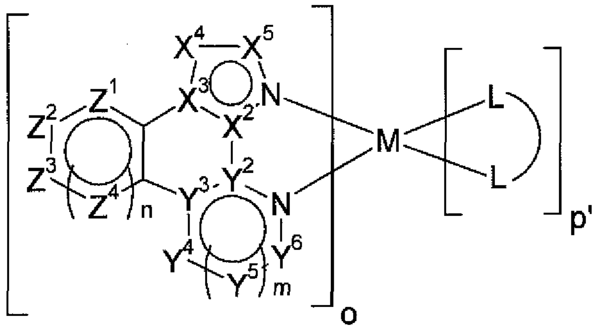

(IIa)

in which

M is a metal atom selected from the group consisting of transition metals of groups IB, IIB, IIIB, IVB, VB, VIB, VIIB, VIII of the Periodic Table of the Elements (CAS version), in any oxidation state possible for the particular metal atom; preferably Ir(III), Pt(II), more preferably Ir(III);

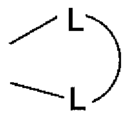

is a bidentate monoanionic ligand;
o is 1, 2, 3 or 4; where o is preferably 1, 2 or 3 when M = Ir(III), more preferably 2 or 3, and is 1 or 2 when M = Pt(II);
p' is 0, 1 or 2; where p' is preferably 0, 1 or 2 when M = Ir(III), more preferably 0 or 1, and is 0 or 1 when M = Pt(II); where p' is the number of ligands

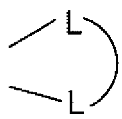
;

where o and p' depend on the oxidation state and coordination number of the metal atom used.

**9.** A metal complex according to claim 8, wherein L in the bidentate monoanionic ligand is in each case independently selected from 0, N and C; preference is given to monoanionic bidentate ligands in which both L groups are O, C or N or one L group is O and the other L group is N or C or one L group is C and the other L group is N; the bidentate monoanionic ligands are more preferably selected from the group consisting of acetylacetonate, picolinate, carbenes, arylpyridines and arylimidazoles, and derivatives of the aforementioned compounds.

**10.** A metal complex according to either of claims 8 and 9, in which:

M is Ir(III) or Pt(II), preferably Ir(III);
o is 1, 2 or 3 when M = Ir(III), preferably 2 or 3; and is 1 or 2 when M = Pt(II);
p' is 0, 1 or 2 when M = Ir(III), preferably 0 or 1; and is 0 or 1 when M = Pt(II);

where the sum of o + p' is 3 when M = Ir(III) and is 2 when M = Pt(II), and o is at least 1.

**11.** An organic light-emitting diode comprising at least one metal complex comprising at least one ligand of the general formula (I)

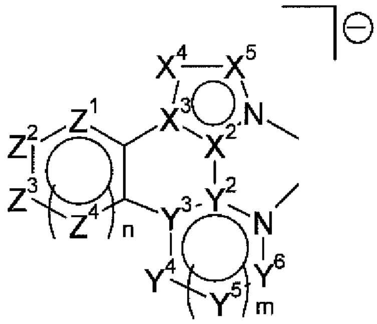

(I)

in which the symbols are each defined as follows:

$Y^5$, $Z^4$ are each independently $CR^1$, CH or N;
$X^2$, $X^3$, $Y^2$, $Y^3$ are each independently N or C;
$Z^4$, $Z^5$ are each independently $CR^1$, CH or N;
$Y^4$, $Y^6$ are each independently $CR^1$, CH or N, where $Y^4$ or $Y^6$, in the case that m = 0, may additionally be $NR^1$, S or O;
$Z^1$, $Z^2$, $Z^3$ are each independently $CR^1$, CH or N; where $Z^1$, $Z^2$ or $Z^3$, in the case that n = 0, may additionally be $NR^1$, S or O;
$R^1$ is independently unsubstituted or substituted alkyl, unsubstituted or substituted cycloalkyl, unsubstituted or substituted heterocycloalkyl., unsubstituted or substituted aryl, unsubstituted or substituted heteroaryl, unsubstituted or substituted alkenyl, unsubstituted or substituted cycloalkenyl, unsubstituted or substituted alkynyl, $SiR^2_3$, halogen, a substituent with donor or acceptor action; in addition, two $R^1$ radicals together may form an alkylene or arylene bridge;
$R^2$ is independently unsubstituted or substituted alkyl, unsubstituted or substituted aryl or unsubstituted or substituted heteroaryl;
m, n are each independently 0 or 1, where the $Z^4$ and $Y^5$ groups are absent when, respectively, n and m = 0.

**12.** A light-emitting layer comprising at least one metal complex comprising at least one ligand of the general formula (I)

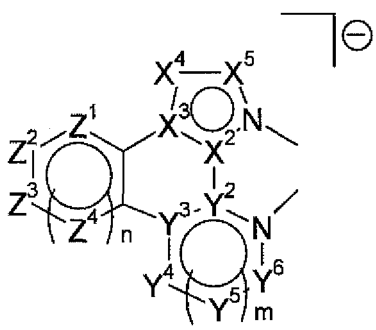

(I)

in which the symbols are each defined as follows:

$Y^5$, $Z^4$ are each independently $CR^1$, CH or N;
$X^2$, $X^3$, $Y^2$, $Y^3$ are each independently N or C;
$X^4$, $X^5$ are each independently $CR^1$, CH or N;
$Y^4$, $Y^6$ are each independently $CR^1$, CH or N, where $Y^4$ or $Y^6$, in the case that m = 0, may additionally be $NR^1$, S or O;
$Z^1$, $Z^2$, $Z^3$ are each independently $CR^1$, CH or N; where $Z^1$, $Z^2$ or $Z^3$, in the case that n = 0, may additionally be

$NR^1$, S or O;

$R^1$ is independently unsubstituted or substituted alkyl, unsubstituted or substituted cycloalkyl, unsubstituted or substituted heterocycloalkyl, unsubstituted or substituted aryl, unsubstituted or substituted heteroaryl, unsubstituted or substituted alkenyl, unsubstituted or substituted cycloalkenyl, unsubstituted or substituted alkynyl, $SiR^2_3$, halogen, a substituent with donor or acceptor action; in addition, two $R^1$ radicals together may form an alkylene or arylene bridge;

$R^2$ is independently unsubstituted or substituted alkyl, unsubstituted or substituted aryl or unsubstituted or substituted heteroaryl;

m, n are each independently 0 or 1, where the $Z^4$ and $Y^5$ groups are absent when, respectively, n and m = 0.

**13.** An organic light-emitting diode comprising at least one light-emitting layer according to claim 12.

**14.** The use of at least one metal complex comprising at least one ligand of the general formula (I)

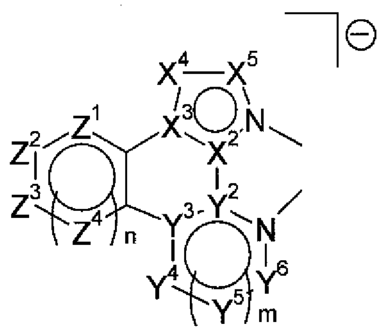

(I)

in which the symbols are each defined as follows:

$Y^5$, $Z^4$ are each independently $CR^1$, CH or N;

$X^2$, $X^3$, $Y^2$, $Y^3$ are each independently N or C;

$X^4$, $X^5$ are each independently $CR^1$, CH or N;

$Y^4$, $Y^6$, are each independently $CR^1$, CH or N, where $Y^4$ or $Y^6$, in the case that m = 0, may additionally be $NR^1$, S or O;

$Z^1$, $Z^2$, $Z^3$ are each independently $CR^1$, CH or N; where $Z^1$, $Z^2$ or $Z^3$, in the case that n = 0, may additionally be $NR^1$, S or O;

$R^1$ is independently unsubstituted or substituted alkyl, unsubstituted or substituted cycloalkyl, unsubstituted or substituted heterocycloalkyl, unsubstituted or substituted aryl, unsubstituted or substituted heteroaryl, unsubstituted or substituted alkenyl, unsubstituted or substituted cycloalkenyl, unsubstituted or substituted alkynyl, $SiR^2_3$, halogen, a substituent with donor or acceptor action; in addition, two $R^1$ radicals together may form an alkylene or arylene bridge;

$R^2$ is independently unsubstituted or substituted alkyl, unsubstituted or substituted aryl or unsubstituted or substituted heteroaryl;

m, n are each independently 0 or 1, where the $Z^4$ and $Y^5$ groups are absent when, respectively, n and m = 0,

in organic light-emitting diodes.

**15.** A device selected from the group consisting of stationary visual display units such as visual display units of computers, televisions, visual display units in printers, kitchen appliances and advertising panels, illuminations, information panels and mobile visual display units such as visual display units in cellphones, laptops, digital cameras, vehicles, and destination displays on buses and trains, comprising at least one organic light-emitting diode according to claim 11 or 13.

**Revendications**

**1.** Complexe métallique contenant au moins un

ligand de formule générale (I)

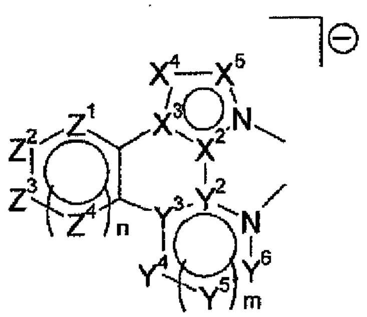

(I)

où les symboles présentent les significations suivantes :

$Y^5$, $Z^4$ indépendamment l'un de l'autre, $CR^1$, CH ou N ;
$X^2$, $X^3$, $Y^2$, $Y^3$ indépendamment l'un de l'autre, N ou C ;
$X^4$, $X^5$ indépendamment l'un de l'autre, $CR^1$, CH ou N ;
$Y^4$, $Y^6$ indépendamment l'un de l'autre, $CR^1$, CH ou N, $Y^4$ ou $Y^6$, dans le cas où m = 0, pouvant en outre signifier $NR^1$, S ou O ;
$Z^1$, $Z^2$, $Z^3$ indépendamment l'un de l'autre, $CR^1$, CH ou N ; $Z^1$, $Z^2$ ou $Z^3$, dans le cas où n = 0, pouvant en outre signifier $NR^1$, S ou O ;
$R^1$ indépendamment l'un de l'autre, alkyle non substitué ou substitué, cycloalkyle non substitué ou substitué, hétérocycloalkyle non substitué ou substitué, aryle non substitué ou substitué, hétéroaryle non substitué ou substitué, alcényle non substitué ou substitué, cycloalcényle non substitué ou substitué, alcynyle non substitué ou substitué, $SiR^2_3$, halogène, un substituant présentant un effet donneur ou accepteur choisi parmi les radicaux alcoxy ou aryloxy, les radicaux ester, les groupes amino, les radicaux amide, les groupes $CH_2F$, les groupes $CHF_2$, les groupes $CF_3$, les groupes CN, les groupes thio, les groupes acide sulfonique, les groupes ester d'acide sulfonique, les groupes acide boronique, les groupes ester de l'acide boronique, les groupes acide phosphonique, les groupes ester de l'acide phosphonique, les radicaux phosphine, les radicaux sulfoxyde, les radicaux sulfonyle, les radicaux sulfure, $SR^3$, les groupes nitro, OCN, les radicaux borane, les radicaux stannate, les groupes imino, les radicaux hydrazine, les radicaux hydrazone, les radicaux oxime, les groupes nitroso, les groupes diazo, les groupes phosphinoxyde, les groupes hydroxy ou les groupes SCN, de plus, deux radicaux $R^1$ peuvent former ensemble un pont alkylène ou arylène ;
$R^2$ indépendamment l'un de l'autre, alkyle non substitué ou substitué, aryle non substitué ou substitué ou hétéroaryle non substitué ou substitué ;
m, n indépendamment l'un de l'autre, 0 ou 1, les groupes $Z^4$ ou, selon le cas, $Y^5$ pour n ou, selon le cas, m = 0 n'étant pas présents.

2. Complexe métallique selon la revendication 1, **caractérisé en ce que** m = 1.

3. Complexe métallique selon la revendication 1 ou 2, **caractérisé en ce que** n = 1.

4. Complexe métallique selon l'une quelconque des revendications 1 à 3, **caractérisé en ce que** $X^4$, $X^5$, $Y^5$, $Y^6$, $Z^1$, $Z^2$, $Z^3$, $Z^4$ indépendamment l'un de l'autre, signifient $CR^1$, CH ou N ;

$Y^4$ signifie $CR^1$, CH ou N ; $Y^4$, dans le cas où m = 0, pouvant en outre signifier $NR^1$, S ou O ;
$X^3$, $Y^3$ signifient N ou C, de préférence C ;
$X^2$, $Y^2$ signifient N ou C, de préférence C.

5. Complexe métallique selon la revendication 3 ou 4, **caractérisé en ce que**

$Z^1$, $Z^2$, $Z^3$, $Z^4$ indépendamment l'un de l'autre, signifient $CR^1$, CH ou N, 0, 1 ou 2 groupes $Z^1$, $Z^2$, $Z^3$, $Z^4$ signifiant de préférence N.

6. Complexe métallique selon l'une quelconque des revendications à 4, **caractérisé en ce que**

$Y^4$, $Y^5$, $Y^6$ indépendamment l'un de l'autre, signifient CH ou $CR^1$ ; et
$Y^2$, $Y^3$ signifient C.

**7.** Complexe métallique selon l'une quelconque des revendications 1 à 6, **caractérisé en ce que** le complexe métallique présente la formule générale (II)

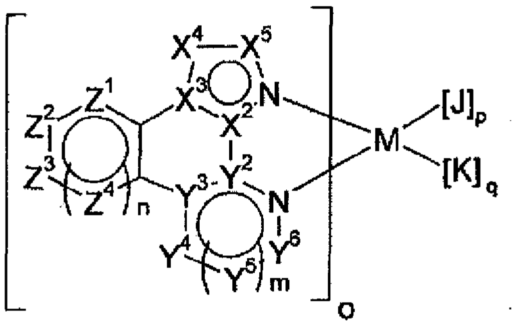

(II)

où :

M signifie un atome métallique choisi dans le groupe constitué par les métaux de transition du groupe IB, IIB, IIIB, IVB, VB, VIB, VIIB, VIII du système périodique des éléments (version CAS), dans chaque étage d'oxydation possible pour l'atome métallique correspondant ; de préférence Ir (III), Pt (II) ou Os (II), de manière particulièrement préférée Ir (III) ;
J signifie un ligand monoanionique ou dianionique, qui peut être monodentate ou bidentate, de préférence un ligand bidentate, monoanionique ;
K signifie un ligand monodentate ou bidentate, neutre, qui n'est généralement pas photoactif : des ligands K préférés sont les phosphines, en particulier les trialkylphosphines, par exemple $PEt_3$, $PnBu_3$, les triarylphosphines, par exemple $PPh_3$ ; les phosphonates et leurs dérivés, les arséniates et leurs dérivés, les phosphites, CO, les nitriles, les amines, les diènes, qui peuvent former un complexe $\pi$ avec M, par exemple 2,4-hexadiène, $\eta^4$-cyclooctadiène et $\eta^2$-cyclooctadiène (à chaque fois 1,3 et 1,5), allyle, méthallyle, cyclooctène, norbornadiène et biscarbènes neutres,
o vaut 1, 2, 3 ou 4 ; o valant de préférence, pour M = Ir (III), 1, 2 ou 3, de manière particulièrement préférée 2 ou 3 et, pour M = Pt (II) ou Os (II), 1 ou 2 ;
p vaut 0, 1, 2, 3 ou 4 ; p valant de préférence, pour M = Ir (III), 0, 1, 2, 3 ou 4, de manière particulièrement préférée 0 ou 2 et, pour M = Pt (II) et Os (II), 0, 1 ou 2, de manière particulièrement préférée 0 ou 2 pour M = Pt (II) et de manière particulièrement préférée 0 pour M = Os (II), p signifiant le nombre de sites de liaison avec le métal M, c'est-à-dire que lorsque p = 2, il peut s'agir de deux ligands monodentates ou d'un ligand bidentate ;
q vaut 0, 1, 2, 3 ou 4 ; q valant de préférence, pour M = Ir (III), 0, 1 ou 2, de manière particulièrement préférée 0 ; pour M = Pt (II), 0 ou 1 de manière particulièrement préférée 0, et, pour Os (II), 2 ou 3, de manière particulièrement préférée 2, q signifiant le nombre de sites de liaison avec le métal M, c'est-à-dire que lorsque q = 2, il peut s'agir de deux ligands monodentates ou d'un ligand bidentate ;

o, p et q dépendant de l'étage d'oxydation et du nombre de coordination de l'atome de métal utilisé et de la charge du ligand.

**8.** Complexe métallique selon la revendication 7, **caractérisé en ce que** le complexe métallique présente la formule générale (IIa)

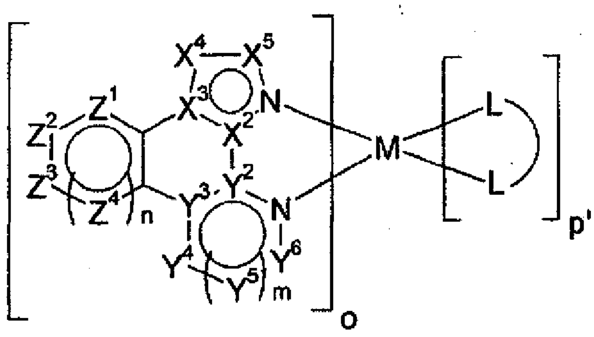 (IIa)

où

M signifie un atome métallique choisi dans le groupe constitué par les métaux de transition du groupe IB, IIB, IIIB, IVB, VB, VIB, VIIB, VIII du système périodique des éléments (version CAS), dans chaque étage d'oxydation possible pour l'atome métallique correspondant ; de préférence Ir (III) ou Pt (II), de manière particulièrement préférée Ir (III) ;

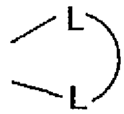

signifie un ligand bidentate monoanionique ;
o vaut 1, 2, 3 ou 4 ; o valant de préférence, pour M = Ir (III), 1, 2 ou 3, de manière particulièrement préférée 2 ou 3 et, pour M = Pt (II), 1 ou 2 ;
p' vaut 0, 1 ou 2 ; p' valant de préférence, pour M = Ir (III), 0, 1 ou 2, de manière particulièrement préférée 0 ou 1 et, pour M = Pt (II), 0 ou 1 ; p' signifiant le nombre de ligands

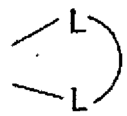

o et p' dépendant de l'étage d'oxydation et du nombre de coordination de l'atome de métal utilisé.

**9.** Complexe métallique selon la revendication 8, **caractérisé en ce que** L dans le ligand monoanionique bidentate est à chaque fois choisi indépendamment l'un de l'autre parmi O, N et C ; on préfère des ligands bidentates monoanioniques bidentates, dans lesquels les deux groupes L signifient 0, C ou N ou un groupe L signifie 0 et l'autre groupe L signifie N ou C ou un groupe L signifie C et l'autre groupe L signifie N ; on préfère en particulier les ligands monoanioniques bidentates choisis dans le groupe constitué par l'acétylacétonate, le picolinate, les carbènes, les arylpyridines et les arylimidazoles et les dérivés des composés susmentionnés.

**10.** Complexe métallique selon l'une quelconque des revendications 8 à 9, où

M signifie Ir (III) ou Pt (II), de préférence Ir (III) ;
o pour M = Ir (III), vaut 1, 2 ou 3, de préférence 2 ou 3 ; pour M = Pt (II), 1 ou 2 ;
p' pour M = Ir (III), vaut 0, 1 ou 2, de préférence 0 ou 1 ; pour M = Pt (II), 0 ou 1 ; la somme o + p' pour M = Ir (III) valant 3 et pour M = Pt (II) 2 et o valant au moins 1.

**11.** Diode lumineuse organique contenant au moins un complexe métallique contenant au moins un ligand de formule générale (I)

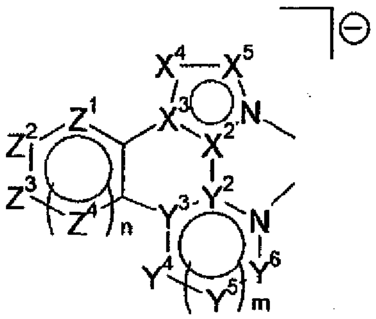

(I)

où les symboles présentent les significations suivantes :

$Y^5$, $Z^4$ indépendamment l'un de l'autre, $CR^1$, CH ou N ;
$X^2$, $X^3$, $Y^2$, $Y^3$ indépendamment l'un de l'autre, N ou C ;
$X^4$, $X^5$ indépendamment l'un de l'autre, $CR^1$, CH ou N ;
$Y^4$, $Y^6$ indépendamment l'un de l'autre, $CR^1$, CH ou N, $Y^4$ ou $Y^6$, dans le cas où m = 0, pouvant en outre signifier $NR^1$, S ou O ;
$Z^1$, $Z^2$, $Z^3$ indépendamment l'un de l'autre, $CR^1$, CH ou N ; $Z^1$, $Z^2$ ou $Z^3$, dans le cas où n = 0, pouvant en outre signifier $NR^1$, S ou O ;
$R^1$ indépendamment l'un de l'autre, alkyle non substitué ou substitué, cycloalkyle non substitué ou substitué, hétérocycloalkyle non substitué ou substitué, aryle non substitué ou substitué, hétéroaryle non substitué ou substitué, alcényle non substitué ou substitué, cycloalcényle non substitué ou substitué, alcynyle non substitué ou substitué, $SiR^2_3$, halogène, un substituant présentant un effet donneur ou accepteur ; de plus, deux radicaux $R^1$ peuvent former ensemble un pont alkylène ou arylène ;
$R^2$ indépendamment l'un de l'autre, alkyle non substitué ou substitué, aryle non substitué ou substitué ou hétéroaryle non substitué ou substitué ;
m, n indépendamment l'un de l'autre, 0 ou 1, les groupes $Z^4$ ou, selon le cas, $Y^5$ pour n ou, selon le cas, m = 0 n'étant pas présents.

**12.** Couche émettant de la lumière contenant au moins un complexe métallique contenant au moins un ligand de formule générale (I)

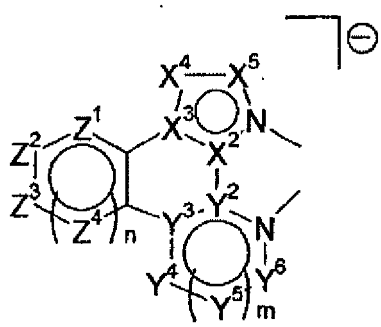

(I)

où les symboles présentent les significations suivantes :

$Y^5$, $Z^4$ indépendamment l'un de l'autre, $CR^1$, CH ou N ;
$X^2$, $X^3$, $Y^2$, $Y^3$ indépendamment l'un de l'autre, N ou C ;
$X^4$, $X^5$ indépendamment l'un de l'autre, $CR^1$, CH ou N ;
$Y^4$, $Y^6$ indépendamment l'un de l'autre, $CR^1$, CH ou N, $Y^4$ ou $Y^5$, dans le cas où m = 0, pouvant en outre signifier $NR^1$, S ou O ;
$Z^1$, $Z^2$, $Z^3$ indépendamment l'un de l'autre, $CR^1$, CH ou N ; $Z^1$, $Z^2$ ou $Z^3$, dans le cas où n = 0, pouvant en outre signifier $NR^1$, S ou O ;
$R^1$ indépendamment l'un de l'autre, alkyle non substitué ou substitué, cycloalkyle non substitué ou substitué,

hétérocycloalkyle non substitué ou substitué, aryle non substitué ou substitué, hétéroaryle non substitué ou substitué, alcényle non substitué ou substitué, cycloalcényle non substitué ou substitué, alcynyle non substitué ou substitué, $SiR^2{}_3$, halogène, un substituant présentant un effet donneur ou accepteur ; de plus, deux radicaux $R^1$ peuvent former ensemble un pont alkylène ou arylène ;
$R^2$ indépendamment l'un de l'autre, alkyle non substitué ou substitué, aryle non substitué ou substitué ou hétéroaryle non substitué ou substitué ;
m, n indépendamment l'un de l'autre, 0 ou 1, les groupes $Z^4$ ou, selon le cas, $Y^5$ pour n ou, selon le cas, m = 0 n'étant pas présents.

**13.** Diode lumineuse contenant au moins une couche émettant de la lumière selon la revendication 12.

**14.** Utilisation d'au moins un complexe métallique contenant au moins un ligand de formule générale (I)

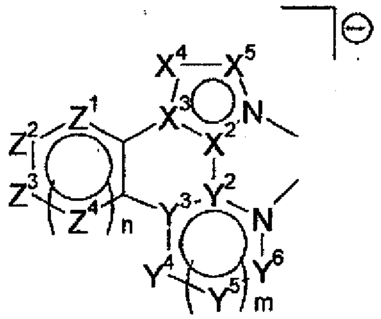

(I)

où les symboles présentent les significations suivantes :

$Y^5$, $Z^4$ indépendamment l'un de l'autre, $CR^1$, CH ou N ;
$X^2$, $X^3$, $Y^2$, $Y^3$ indépendamment l'un de l'autre, N ou C ;
$X^4$, $X^5$ indépendamment l'un de l'autre, $CR^1$, CH ou N ;
$Y^4$, $Y^6$ indépendamment l'un de l'autre, $CR^1$, CH ou N, $Y^4$ ou $Y^6$, dans le cas où m = 0, pouvant en outre signifier $NR^1$, S ou O ;
$Z^1$, $Z^2$, $Z^3$ indépendamment l'un de l'autre, $CR^1$, CH ou N ; $Z^1$, $Z^2$ ou $Z^3$, dans le cas où n = 0, pouvant en outre signifier $NR^1$, S ou O ;
$R^1$ indépendamment l'un de l'autre, alkyle non substitué ou substitué, cycloalkyle non substitué ou substitué, hétérocycloalkyle non substitué ou substitué, aryle non substitué ou substitué, hétéroaryle non substitué ou substitué, alcényle non substitué ou substitué, cycloalcényle non substitué ou substitué, alcynyle non substitué ou substitué, $SiR^2{}_3$, halogène, un substituant présentant un effet donneur ou accepteur ; de plus, deux radicaux $R^1$ peuvent former ensemble un pont alkylène ou arylène ;
$R^2$ indépendamment l'un de l'autre, alkyle non substitué ou substitué, aryle non substitué ou substitué ou hétéroaryle non substitué ou substitué ;
m, n indépendamment l'un de l'autre, 0 ou 1, les groupes $Z^4$ ou, selon le cas, $Y^5$ pour n ou, selon le cas, m = 0 n'étant pas présents,

dans des diodes lumineuses organiques.

**15.** Dispositif choisi dans le groupe constitué par les écrans stationnaires, tels que les écrans d'ordinateurs, de téléviseurs, les écrans dans les imprimantes, les appareils de cuisine ainsi que les panneaux publicitaires, les éclairages, les panneaux indicateurs et les écrans mobiles, tels que les écrans dans les téléphones portables, les ordinateurs portables, les caméras digitales, les véhicules ainsi que les affichages de destination sur les bus et les trains contenant au moins une diode lumineuse selon la revendication 11 ou 13.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2005113704 A
- WO 2007095118 A
- WO 2008000726 A
- WO 2005019373 A
- WO 20050113704 A
- WO 2006018292 A
- WO 2006056418 A
- WO 2007115981 A
- WO 2007115970 A
- WO 2008000727 A
- WO 2006067074 A
- WO 2006106842 A
- WO 2007018067 A
- WO 2007058255 A
- WO 2007069542 A
- US 2007108891 A
- WO 2007058080 A
- WO 2007058104 A
- WO 0215645 A
- WO 2005123873 A
- US 2007196690 A
- WO 2006121811 A
- EP 2007059648 W
- WO 2004095889 A
- EP 1617710 A
- EP 1617711 A
- WO 2006112265 A
- WO 2006130598 A


### In der Beschreibung aufgeführte Nicht-Patentliteratur


- **M. A. BALDO et al.** *Appl. Phys. Lett.,* 1999, vol. 75, 4-6
- **H. MENG ; N. HERRON.** Organic Small Molecule Materials for Organic Light-Emitting Devices in Organic Light-Emitting Materials and Devices. Taylor & Francis, 2007, 295-411
- *Nature,* 11. Juni 1992, vol. 357, 477-479
- Kirk-Othmer Encyclopedia of Chemical Technologie. 1996, vol. 18, 837-860
- **W. GAO ; A. KAHN.** *J. Appl. Phys.,* 01. Juli 2003, vol. 94 (1
- **A. G. WERNER ; F. LI ; K. HARADA ; M. PFEIFFER ; T. FRITZ ; K. LEO.** *Appl. Phys. Lett.,* 23. Juni 2003, vol. 82 (25
- **PFEIFFER et al.** *Organic Electronics,* 2003, vol. 4, 89-103